# EUROPÄISCHE PATENTSCHRIFT

(11) **EP 1 355 674 B1**
(45) Veröffentlichungstag und Bekanntmachung des Hinweises auf die Patenterteilung: **27.10.2010**
(21) Anmeldenummer: 02708308.8
(22) Anmeldetag: 23.01.2002
(51) Int. Cl.: A61K 47/48

(54) **OLIGO- ODER POLYALKYLENGLYCOLGEKOPPELTE THROMBININHIBITOREN**
OLIGO ALKYLENE GLYCOL OR POLYALKYLENE GLYCOL-COUPLED THROMBIN INHIBITORS
INHIBITEURS DE THROMBINE A COUPLAGE OLIGO ALKYLENE-GLYCOL OU POLYALKYLENE-GLYCOL

(30) Priorität: 23.01.2001 DE 10102878
(43) Veröffentlichungstag der Anmeldung: 29.10.2003
(73) Patentinhaber: JenAffin GmbH, 07745 Jena (DE)
(72) Erfinder: STEINMETZER, Torsten, 07743 Jena (DE); NOWAK, Götz, 99097 Erfurt (DE)
(74) Vertreter: Vossius & Partner
(86) Internationale Anmeldenummer: PCT/EP2002/000652
(87) Internationale Veröffentlichungsnummer: WO 2002/059065

(56) Entgegenhaltungen:
- EP-A- 0 658 585
- WO-A-91/08229
- WO-A-98/32466
- US-A- 5 583 146
- US-A- 5 914 319
- ZALIPSKY S: "CHEMISTRY OF POLYETHYLENE GLYCOL CONJUGATES WITH BIOLOGICALLY ACTIVE MOLECULES" ADVANCED DRUG DELIVERY REVIEWS, AMSTERDAM, NL, Bd. 16, Nr. 2/3, 1995, Seiten 157-182, XP002037428 ISSN: 0169-409X
- LORAND LASZLO ET AL: "A double-headed Gly-Pro-Arg-Pro ligand mimics the functions of the E domain of fibrin for promoting the end-to-end crosslinking of gamma chains by factor XIIIa." PROCEEDINGS OF THE NATIONAL ACADEMY OF SCIENCES OF THE UNITED STATES, Bd. 95, Nr. 2, 20. Januar 1998 (1998-01-20), Seiten 537-541, XP002206582 Jan. 20, 1998 ISSN: 0027-8424
- STEINMETZER, TORSTEN ET AL: "New thrombin inhibitors based on D-Cha-pro-derivatives" JOURNAL OF ENZYME INHIBITION (1999), 14(3), 203-216 , XP001084488

## Beschreibung

Die Erfindung betrifft neuartige, kovalente Oligo- und Polyalkylenglykolkonjugate mit synthetischen Inhibitoren trypsinartiger Serinproteasen, insbesondere Inhibitoren der Blutgerinnungsprotease Thrombin, synthetische Zwischenstufen zu deren Herstellung und ihre Verwendung zur Herstellung von Wirkstoffen für die Therapie oder Prophylaxe thrombotischer Komplikationen.

Die trypsinartige Serinprotease Thrombin ist das zentrale Enzym der Blutgerinnungskaskade. Unter anderem spaltet es Fibrinogen, wodurch ein Fibringerinnsel entsteht, dass durch folgende Quervernetzung, katalysiert durch Faktor XIIIa, stabilisiert wird (Davie et al., 1991 Biochemistry30, 10363-10370). Auch der Faktor XIIIa selbst wird durch die katalytische Wirkung des Thrombins aus seiner Zymogenform aktiviert. Thrombin kann die Blutgerinnungskaskade zusätzlich durch die Aktivierung der Faktoren V und VIII beschleunigen. Weiterhin ist Thrombin in der Lage, den auf der Thrombocytenoberfläche befindlichen Thrombinrezeptor spezifisch zu stimulieren, was zu einer Plättchenaktivierung führt Zu hohe Thrombinaktivitäten können zu verschiedenen thrombotischen Komplikationen führen, wie z.B. Myocardinfarkt, tiefer Venenthrombose, peripheren arteriellen Verschlusskrankheiten oder Lungenembolie. Thrombotische Komplikationen können auch bei Therapien auftreten, bei denen Blut in Kontakt mit nichtphysiologischen Oberflächen kommt, wie z.B. bei operativen Eingriffen, bei der Hämodialyse oder in Herz-Lungenmaschinen.
Durch die Verwendung des Thrombininhibitors Hirudin konnte nachgewiesen werden, daß spezifische Thrombinhemmer blutgerinnungshemmende Eigenschaften besitzen und für die Vermeidung thrombotischer Komplikationen geeignet sind (Markwardt, 1970 Methods in Enzymol. 19, 924-932). Hirudin ist ein natürlich vorkommendes Protein, das aus dem medizinischen Blutegel isoliert wurde und seit einigen Jahren in rekombinanter Form vorliegt. Inzwischen wurde rekombinant hergestelltes Hirudin für einige Indikationen, wie z.B. zur Vermeidung von Thrombosen nach Hüftgelenksoperationen oder bei Heparin-induzierter Thrombocytopenia, zugelassen (Menear, 1999 Expert Opinion on Investigational Drugs 8, 1373-1384).
Da rekombinante Proteine kostenintensiv hergestellt werden müssen, nur parenteral appliziert werden können und körperfremde Proteine eine antigene Wirkung besitzen, wird intensiv an der Entwicklung niedermolekularer, synthetischer Thrombininhibitorn zur Verwendung als oral wirksame Antikoagulantien geforscht. Trotz intensiver Bemühungen sind bisher nur zwei niedermolekulare Thrombininhibitoren, Argatroban und Gabexate Mesilate, in Japan zugelassen, jedoch sind auch diese Verbindungen nur parenteral wirksam und besitzen eine sehr kurze Verweildauer im Blut. Argatroban erhielt im Jahr 2000 auch die Zulassung in den USA.

Aus der Literatur ist bekannt, dass man durch kovalente Konjugation mit Makromolekülen, wie z.B. Polysacchariden oder Polyethylenglykolen (PEG), die Verweildauer von Proteinen, Inhibitoren und anderen Wirkstoffen im Blut verlängern kann. Dies gilt z.B. auch für ein PEG-gekoppeltes Hirudin (Kurfürst et al., WO 91/08229) oder einen PEG-gekoppelten, niedermolekularen Thrombinhemmstoff basierend auf der CRC-220 (Stüber et al. Peptide Research 8, 78-85, (1995); Stüber und Koschinsky EP 0658 585 A2). Beide PEG-Inhibitoren besitzen eine längere Halbwertszeit im Blut von Versuchstieren im Vergleich zu den freien, unmodifizierten Verbindungen.
Aus der Entwicklung niedermolekularer Thrombinhemmstoffe ist bekannt, dass man die für die Bindung notwendigen basischen Aminosäuren Arginin und Lysin, als auch deren Mimetika 4-Amidinophenylglycin oder 3- und 4-Amidinophenylalanin durch geeignete decarboxylierte Analoga ersetzen kann, ohne dass ein Verlust an Thrombinaffinität auftritt. Beispiele dafür sind Verbindungen mit C-terminalem Agmatin (D-Phe-Pro-Agmatin; Bajusz et al., 1982 Folia Haematol., Leipzig 109, 16-21), Noragmatin (Inogatran; Teger-Nilsson, WO 93/11152), 4-Amidinobenzylamin (Melagatran, Antonsson et al., WO 94/29336 oder Böhm et al., US 5,852,051) oder 4-Aminomethyl-N(Amidino)Piperidin Sanderson et al., 1997 Bioorganic & Medicinal Chemistry Letters 7, 1497-1500).

Ein Problem bei der Anwendung von Thrombininhibitoren ist oftmals, dass die Leber diese Stoffe rasch aufnimmt, metabolisiert und sezerniert. Dabei können neue Metabolite mit unerwünschten Nebenwirkungen entstehen, die teilweise wieder in den Blutkreislauf abgegeben werden und so z.B: für eine Medikamentenentwicklung nur bedingt geeignet sind. Eine Ausnahme in die Leber wurde z. B. für Thrombininhibitoren aus völlig verschiedenen Substanzklassen gezeigt, wie z.B. für CRC220, TAPAP, NAPAP, Argatroban oder Efegatran.

Überraschenderweise konnten wir feststellen, dass die hier beschriebenen Oligo- oder Polyalkylenglykol-gebundenen Inhibitoren (Inhibitor-Konjugate) in verstärktem Maß bzw. nahezu vollständig über die Niere eliminiert werden. Das heißt, dass die kovalente Kopplung eines Oligo- oder Polyalkylenglykolanteils an die von uns verwendeten niedermolekularen, synthetischen Thrombininhibitoren deren Ausscheidungsweg verändert. Gleichzeitig erhöht sich die Verweildauer der Inhibitor-Konjugate im Blut im Vergleich zu den freien Inhibitoren und damit auch ihre gerinnungshemmende Wirkung, die strikt von der Konzentration im Blut abhängig ist (Hauptmann und Stürzebecher, 1999 Thrombosis Research 93, 203-241).
Unter der Bezeichnung Inhibitor werden im Rahmen der vorliegenden Anmeldung Moleküle verstanden die spezifisch an das aktive Zentrum des Zielenzyms binden und so zu dessen Hemmung führen. Werden solche Moleküle zusammen mit Oligo- oder Polyalkylenglykolen in die erfindungsgemäßen Inhibitorkonjugate integriert, so bilden sie die Inhibitorstruktur, d.h. den Teil der erfindungsgemäßen Konjugate, dessen Struktur eine spezifische Wechselwirkung mit dem Zielenzym erlaubt. Als bivalente Inhibitorkonjugate werden solche bezeichnet, deren Oligo- oder Polyalkylenanteil mit zwei Inhibitorstruktwen verknüpft ist

Wir haben gefunden, dass man durch Einbau von decarboxylierten, basischen P1-Resten in Oligo- und Polyalkylenglykol-gekoppelte Verbindungen hochpotente Thrombininhibitor-Konjugate erhalten kann. Zusätzlich haben wir gefunden, dass man auch Thrombininhibitoren mit Oligo- und Polyalkylenglykolen modifizieren kann, die als Pl-Rest substituierte Arginylketonderivate besitzen. Bei Einsatz solcher Verbindungen ist man weder auf das im aus dem Stand der Technik bekannten PEG-gekoppelten CRC-220 verwendete, kostenintensive D-4-Amidinophenylalanin noch auf nur unter großem Aufwand herzustellendes rekombinantes Hirudin angewiesen.

Diese Oligo- und Polyalkylenglykol-gekoppelten Inhibitoren werden im Gegensatz zu den freien Inhibitoren nicht bzw. nur in geringem Umfang in die Leberzellen aufgenommen und dort verstoffwechselt oder in die Galle überführt, sondern fast ausschließlich über die Niere eliminiert. Nach subkutaner Applikation der Oligo- und Polyälkylenglykol-gekoppelten Inhibitoren wurden lang anhaltende und für eine Gerinnungshemmung notwendige Blutspiegel der Inhibitoren bestimmt und es wurde festgestellt, das hierfür die stark verzögerte Transferrate der Substanzen zwischen Extravasalraum und Intravasalraum verantwortlich ist. Die gleichmäßige Verteilung nach Resorption aus einem subkutanen Injektionsdepot führt zu einem ausschließlichen Ausfüllen des extrazellulären Wasserraumes, da die Kopplung der Inhibitoren mit Oligo- oder Polyalkylenglykolen zu Substanzen mit vergrößerten Hydrathüllen führt.

Die von uns entwickelten Verbindungen besitzen die allgemeine Struktur (I) 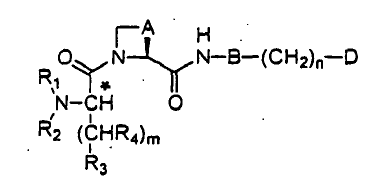 wobei
A eine Ethylengruppe ist
B eine Bindung oder 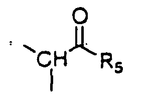 ist, wobei R₅ ein Alkylrest ist der 1 bis 4, bevorzugt 1 oder 2 und besonders bevorzugt ein C-Atom umfasst und mit einem oder mehreren gleichen oder unterschiedlichen Halogenatomen, bevorzugt Cl oder F, oder mit einem Rest 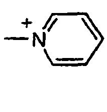 substituiert sein kann; n 0, 1, 2, 3, 4 oder 5 ist,
D einen Rest 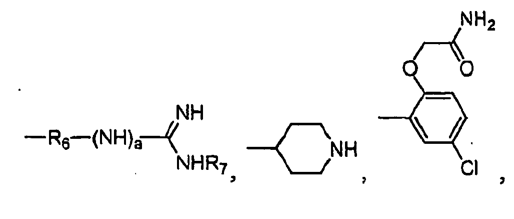 -R₈-(CH₂)_{b}-NH₂, oder 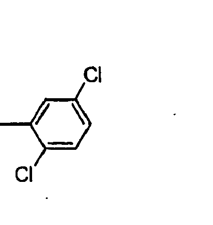 darstellt, wobei R₆ und R₈ unabhängig voneinander bivalente Reste sind, ausgewählt aus aromatischen oder gesättigten 6-Ringen, die neben Kohlenstoff noch ein Heteroatom, bevorzugt ein Stickstoffatom, enthalten können und einen oder mehrere gleiche oder unterschiedliche Alkylsubstituenten tragen können, und R₆ darüberhinaus -NH- sein kann falls a 0 ist, R₇ ein Wassezstoffatom oder -NH₂ ist und a 0 oder 1 und b 0, 1 oder 2 ist;
R₁ ein Wasserstoffatom oder ein Arylsulfonyl-, Aralkylsulfonyl-, Cycloalkylmethylsulfonyl-, Cycloalkylethylsulfonyl- oder ein Alkylsulfonylrest ist, deren Arylteil gegebenenfalls einen, zwei, drei, vier oder fünf Substituenten, die unabhängig voneinander ausgewählt sind aus Halogenatomen, Alkyl- oder Alkoxyresten, tragen kann, oder mit einem weiteren Arylrest verknüpft ist;
und entweder
R₂ -(CH₂)ₚ-CO-NH-(CH₂)_{q}-X, -(CH₂)ᵣ-NH-CO-(CH₂)ₛ-V-X oder
-(CH₂)ᵣ-NH-CO-O-(CH₂)ₛ-V-X ist
wobei
p eine ganze Zahl von 1 bis 5 und q eine ganze Zahl von 2 bis 5 ist,
r eine ganze Zahl von 2 bis 5 und s ist eine ganze Zahl von 1-5 ist,
V eine Bindung oder -CO-NH-(CH₂)_{c}- und c eine ganze Zahl von 2 bis 5 ist, und
X einen Oligo- oder Polyalkylenglykol der Struktur -[O-(CH₂)_{d}]_{c}-OZ oder -[O-CH(CH₃)-CH₂]ₑ-OZ oder einen Cyclus der Struktur 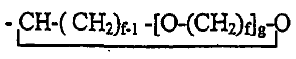 darstellt und d eine ganze Zahl von 2 bis 6, e eine ganze Zahl von 3 bis 1000, Z ein Wasserstoffatom oder ein Alkylrest ist oder die gesamte Inhibitorstruktur repliziert, die an die freie Valenz des Restes X gebunden ist, f eine ganze Zahl von 2 bis 6 ist und g eine ganze Zahl von 3 bis 10, bevorzugt 3-7 ist; und
R₃ ein Phenyl- oder Cyclohexylrest ist, der mit 1 bis 5 gleichen oder unterschiedlichen Substituenten, unabhängig voneinander ausgewählt aus Halogenatomen, Alkyl- oder Alkoxyresten oder Hydroxylgruppen substituiert sein kann und
R₄ ein Wasserstoffatom, Phenyl- oder Cyclohexylrest ist, der mit 1 bis 5 gleichen oder unterschiedlichen Substituenten, unabhängig voneinander ausgewählt aus Halogenatomen, Alkyl- oder Alkoxyresten oder Hydroxylgruppen substituiert sein kann, wobei das Wasserstoffatom bevorzugt wird wenn m größer als 1 ist,
m 0, 1, 2, 3 oder 4 ist und
am mit *gekennzeichneten Kohlenstoffatom eine D-Konfiguration vorliegt;
oder
R₂ ein Wasserstoffatom ist und
R₃ -CO-NH-(CH₂)_{q}-X, -CO-W₁-W₂-(CH₂)_{q}-X, -NH-CO-(CH₂)₅-V-X, -NH-CO-O-(CH₂)ₛ-V-X, -S-CH₂-CO-NH-(CH₂)ₓ-X oder -S-S-CH₂-CH₂-X ist,
wobei q, s X und V wie vorstehend definiert sind und t eine ganze Zahl von 2 bis 5 ist, und der Rest W₁ -O- oder -NH- ist, und W₂ eine Bindung darstellt oder die Struktur -(CH₂)ᵥ-Ph-(CH₂)_{v'}-Amid- aufweist, in der v und v' unabhängig von einander 0, 1 oder 2 sind, Ph einen 1,2-, 1,3- oder 1,4-substituierten Phenylrest und Amid -HN-(O)C- oder-C(O)NH- darstellt, R₄ ein Wasserstoffatom ist,
m 1, 2, 3, 4 oder 5 ist und
am mit * gekennzeichneten Kohlenstoffatom eine L-Konfiguration vorliegt.

Bevorzugt stellt B in der allgemeinen Formel (I) eine Bindung oder eine der folgenden Strukturen dar:

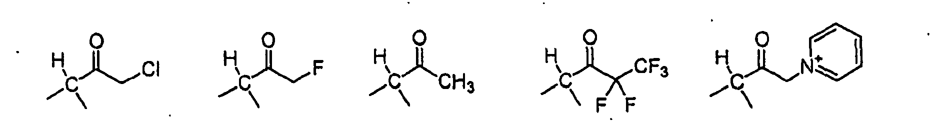

Im Rest D in der allgemeinen Formel (I) ist R₆ bevorzugt 1,4-Piperidindiyl, 1,3-Piperidindiyl, 1,4-Phenylen, 1,3-Phenylen, 1,4-Cyclohexandiyl, 1,3-Cyclohexandiyl oder -NH-. R₈ ist bevorzugt 1,4-Phenylen, 1,3-phenylen, 1,4-Cyclohexandiyl, 1,3-Cyclohexandiyl oder 2,5-Pyridindiyl.

Besonders bevorzugt sind für D die folgenden Strukturen, die über die mit ∼ markierte Bindung mit dem Rest der allgemeinen Struktur (I) verknüpft sind

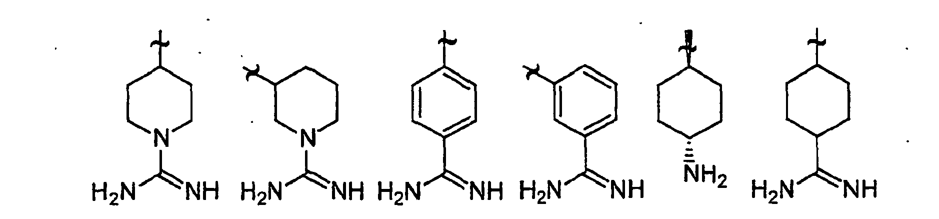

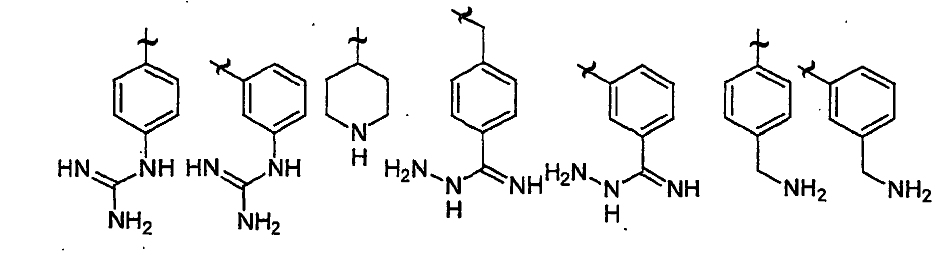

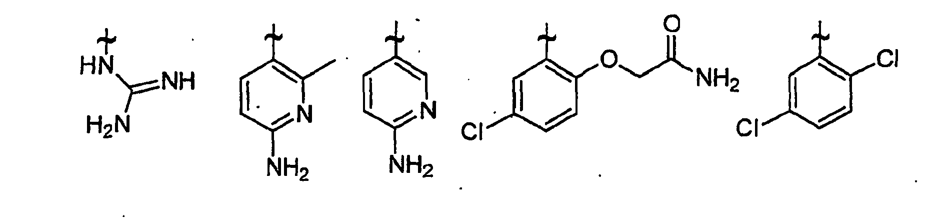

Bevorzugte Substituenten des Arylanteils von R₁ sind Methyl, Ethyl, Propyl, Methoxy oder Ethoxygruppen. Als Halogenatome werden bevorzugt Cl, Br oder F eingesetzt. Bevorzugte Reste R₁ sind damit beispielsweise 3-Methoxy-phenylsulfonyl, 3,4-Dichlor-phenylsulfonyl, Benzylsulfonyl, 3,4-Dimethoxy-phenylsulfonyl, 2,4,5-Trichlor-phenylsulfonyl, 2-Naphtylsulfonyl, 4-Chlor-phenylsulfonyl, Pentamethyl-phenylsulfonyl, 2,4,6-Triisopropyl-phenylsulfonyl. Als besonders bevorzugter Rest R₁ ist ein 4-methoxysubstituierter Phenylsulfonylrest zu nennen, oder ein 4-methoxy-substituierter Phenylsulfonylrest, der an Position 3 zusätzlich mit einem Chloratom oder mit einer Methylgruppe substituiert ist. Er kann auch an den Positionen 2, und/oder 6 gegebenenfalls weitere Methylreste tragen.

Der von X umfasste Oligo- oder Polyalkylenglykolanteil der erfindungsgemäßen Verbindungen wird bevorzugt durch ein Polyethylenglykol oder Polypropylenglykol dargestellt, d.h. die Parameter d und f sind vorzugsweise 2 oder 3. Bevorzugte Kettenlängen ergeben sich aus Werten des Parameters e von 3 und 500, besonders bevorzugt hat das resultierende Polyalkylenglycol ein mittleres Molekulargewicht, das zwischen 750 und 20000 Da liegt, insbesondere um 750, 2000, 3000, 3400, 5000 6000, 10000 oder 20000 Da. Bildet X eine cyclische Struktur, d.h. einen Kronenether der Struktur 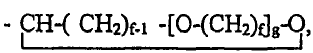 so nimmt f ebenfalls bevorzugt die Werte 2 und 3 an. Besonders bevorzugte Werte für g sind hier 3, 4, 5 oder 6.

Überraschenderweise wurde festgestellt, dass die Wirkung solcher Kronenether der von Polyalkylenglykolketten eines erheblich höheren Molekulargewichtes entspricht So besitzen beispielsweise Kronenetherkonjugate die aus 5 PEG-Einheiten aufgebaut sind eine Eliminierungskinetik im Blut von Ratten, die der von Polyethylenglykol (PEG)-Konjugaten mit einem Molekulargewicht des PEG Anteils von 5000 bis 10000 Da entspricht. Darüber hinaus stellen Kronenether im Vergleich zu linearen Polyalkylenglykolen, die notwendigerweise eine gewisse Breite der Molekulargewichtsverteilung aufweisen, chemisch einheitlich definierte Verbindungen dar, was insbesondere bei der Bereitstellung von Medikamenten Vorteile mit sich bringt

Besitzt X die Struktur -[O-(CH₂)_{d}]ₑ-OZ oder -[O-CH(CH₃)-CH₂]ₑ-OZ und wird Z so gewählt, dass es der Inhibitorstruktur entspricht, an welche der Rest X mit seiner freien Valenz gebunden ist, die sich an dem Ende der Oligo- oder Polyalkylenglykolstruktur befindet, welche Z entgegengesetzt liegt, so entsteht ein zweifach inhibitorfunktionalisiertes Oligo- oder Polyalkylenglykol. Mittels solcher bifunktionalen Konjugate läßt sich die Hemmwirkung bezogen auf die Anzahl der vorhandenen Moleküle der Struktur (I) erhöhen. Dies kann unter anderem bei der Modifizierung von Oberflächen mit Molekülen der Struktur (I) von Vorteil sein, wenn eine möglichst hohe Inhibitordichte pro Flächeneinheit erreicht werden soll.

Soweit nicht gesondert definiert bezieht sich der Begriff "Alkyl-" im Rahmen der vorliegenden Erfindung auf lineare oder verzweigte C₁-C₈ Alkylreste, bevorzugt C₁-C₅, besonders bevorzugt C₁-C₃. Cycloalkylreste beinhalten 3-8, bevorzugt 3 bis 6 C-Atome. "Alkoxy-" bezieht sich auf solche Reste, deren Kohlenstoffkette 1 bis 8, bevorzugt 1 bis 5, besonders bevorzugt 1 bis 3 C-Atome umfasst. "Aralkyl-" beschreibt Strukturen mit 7 bis 19, bevorzugt 7 bis 13, besonders bevorzugt 7 bis 9 C-Atomen. Arylreste sind Reste mit 6 bis 18, bevorzugt 6 bis 12, besonders bevorzugt 6 C-Atomen. Diese Definition gilt unabhängig voneinander für jeden Fall des Auftretens der obigen Bezeichnungen.

Besonders bevorzugt sind im Rahmen der vorliegenden Erfindung die folgenden Verbindungen der Formel (Ia-h) 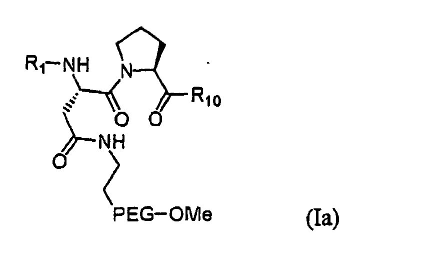 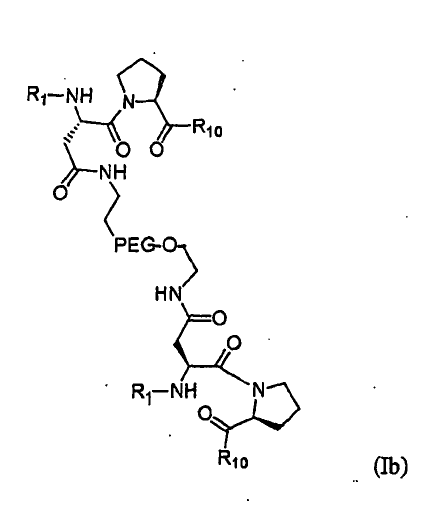 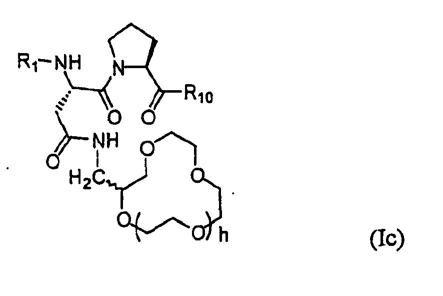 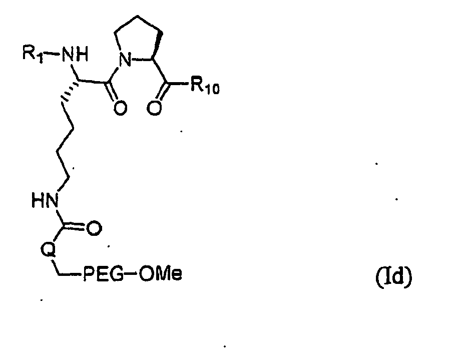 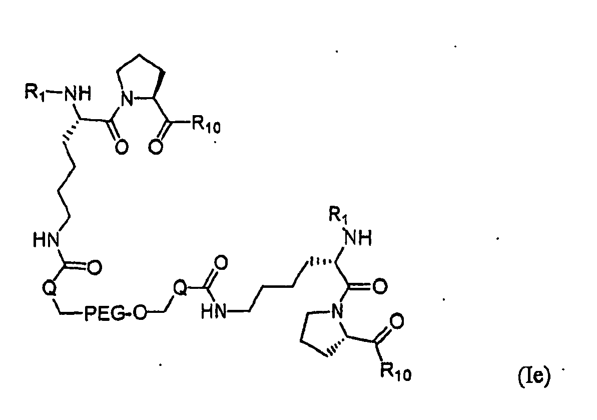 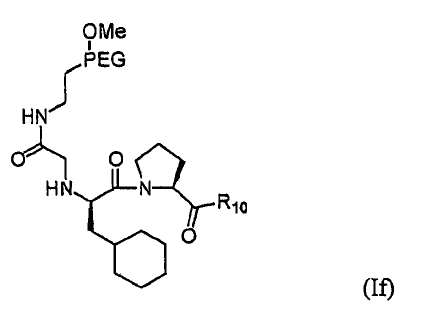 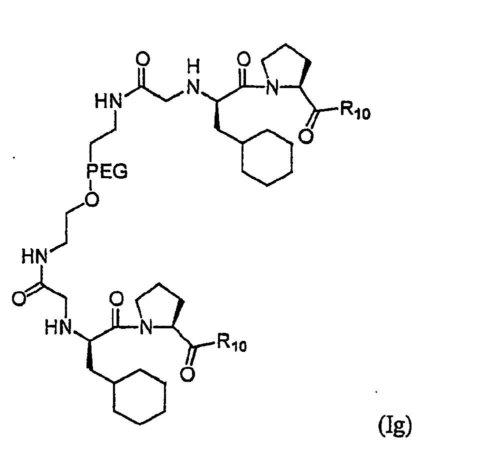 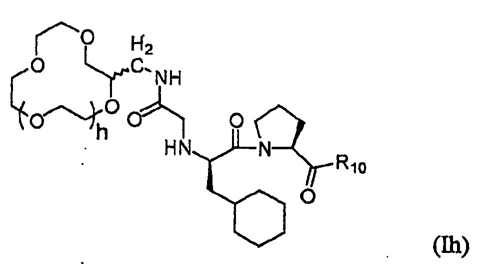 in denen R₁ ein 4-Methoxy-phenylsulfonyl-, 4-Methoxy-3-Chlor-phenylsulfonyl-, 4-Methoxy-3-Methyl-phenylsulfonyl- oder 4-Methoxy-2,3,6-trimethyl-phenylsulfonylrest (Mtr) ist,
Q -(CH₂)s'- mit s' = 0, 1, 2, 3 oder 4, -O-CH₂- oder -CH₂-CH₂-CO-NH-CH₂ ist und
PEG eine Polyethylenglykolstruktur der Formel -[O-C₂H₄]ᵢ- darstellt, mit Werten für i von 3 bis 1000, bevorzugt 3 bis 500, wobei solche Werte für i bevorzugt sind, die ein durchschnittliches Molekulargewicht des PEG von um 750 bis 20000 Da, bevorzugt um 750, 2000, 3000, 3400, 5000, 6000, 10000 oder 2000 Da ergeben. Die Variable h steht für 1, 2, 3 oder 4 und R₁₀ ist

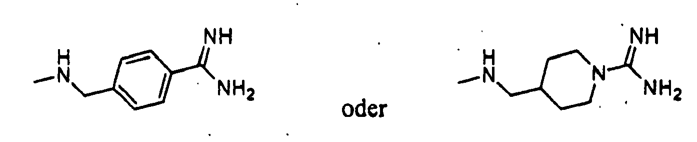

Die Verbindungen der allgemeinen Formel (I) werden in einer pharmazeutisch geeigneten, nicht-toxischen Salzform hergestellt, wie z. B. als Hydrochlorid-, Hydrobromid-, Acetat-, Lactat-; Zitrat-, Tosylat- oder Trifluoracetat-Salz. Sie können in den gebräuchlichen galenischen Applikationsformen fest oder flüssig angewendet werden, z.B. als Lösungen, Sprays, Salben oder Cremes. Diese werden in üblicher Weise hergestellt. Die Wirkstoffe können dabei mit den üblichen galenischen Hilfsmitteln wie Füllstoffen, Konservierungsmitteln, FlieBregulierungsmitteln, Netzmitteln, Dispergiermitteln, Emulgatoren, Lösungsmitteln und/oder Treibgasen verarbeitet werden (vgl. H. Sucker et al., Pharmazeutische Technologie, Thieme-Verlag, Stuttgart, 1978).

Die Verbindungen können in üblicher Weise oral oder parenteral (subkutan, intravenös, intramuskulär, intraperitoneal) verabreicht werden. Sie können für diagnostische Zwecke verwendet werden oder sind auch für biotechnologische Prozesse, wie z.B. Affinitätsreinigungen von Proteasen oder das Entfernen von Proteasen aus Lösungen jeglicher Art geeignet.

Verbindungen der Struktur (I) sind auch besonders für eine Beschichtung oder Modifikation der Oberfläche makromolekularer Träger geeignet, um die Gerinnung an diesen Trägern zu verhindern bzw. zu reduzieren. Diese makromolekularen Träger, insbesondere Oberflächen medizinischer Gegenstände und Geräte wie z.B Hämodialysatoren, Oxygenatoren und deren Schlauchsysteme, bestehen bevorzugt aus Polymethylmethacrylat, Copolymeren mit Polymethylmethacrylat und analogen Kunststoffen, wie sie in WO 98/46648 (Bucha und Nowak) definiert sind. Zum Einsatz kommen hier Homo- oder Copolymere, zu deren Herstellung mindestens ein Monomertyp eingesetzt wird, der neben einer polymerisierbaren Doppelbindung oder einer polykondensierbaren funktionellen Gruppe eine weitere Carbonylgruppe in Form eines Ketons oder eines Carbonsäurederivats enthält, die nicht an der Polymerisationsreaktion teilnimmt. Bevorzugt enthält das Polymer eine wiederkehrende Einheit der Formel (A): 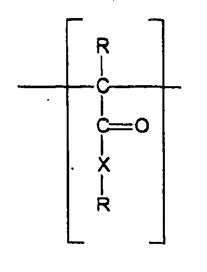 wobei die Reste R gleich oder verschieden sein können und einen Alkyl oder Arylrest oder ein Wasserstoffatom darstellen. Der Alkylrest kann linear oder verzweigt sein und besteht bevorzugt aus 1 bis 20 Kohlenstoffatomen. Der Arylrest besteht bevorzugt aus 6 bis 18, besonders bevorzugt aus 6 bis 12 Kohlenstoffatomen. Der Rest X ist fakultativ und bedeutet O, N oder CH₂. Für den Fall X = N trägt N zusätzlich zu dem in Formel (A) vermerkten einen weiteren Rest R, der unabhängig von den anderen Resten R wie vorstehend definiert ist.

Besonders bevorzugt als Alkylrest ist ein geradkettiger oder verzweigter, gegebenenfalls substituierter C₁₋₈-Alkylrest, beispielsweise ein Methyl-, Ethyl- oder Propylrest. Beispiele für gegebenenfalls vorhandene Substituenten umfassen ein oder mehrere Halogenatome, z.B. Fluor-, Chlor-, Brom- oder Iodatome oder Hydroxylgruppen, C₁₋₆-Alkylreste oder C₁₋₆-Alkoxyreste oder C₁₋₆-Alkylthiolreste. Der Arylrest ist besonders bevorzugt ein monocyclischer oder bicyclischer, gegebenenfalls substituierter Arylrest, der gegebenenfalls ein oder mehrere Heteroatome enthalten kann. Beispiele für solche Arylreste sind Phenyl, 1- oder 2-Naphthyl, Indenyl- oder Isoindenylreste. Beispiele für heteroatomhaltige Arylreste sind C₃₋₉-Heteroarylreste, die Heteroatome, ausgewählt aus Sauerstoff-, Schwefel- oder Stickstoffatomen, enthalten. Monocyclische Heteroarylreste umfassen beispielsweise Pyrolyl-, Furyl-, Thienyl-, Imidazolyl-, N-Methylimidazolyl-, N-Ethylimidazolyl-, Benzothiazolyl-, Chinazolinyl-, Naphthylpyridinyl-, Chinolyinyl-, Isochinolinyl- und Tetrazolylreste.

Ein bevorzugtes Polymer, das solche Gruppen enthält, ist ein Polyalkylmethacrylat (PAMA) mit einem Alkylrest, der vorzugsweise 1-6 C-Atome umfasst, wie z.B. Polymethylmethacrylat (PMMA), Polyethylniethacrylat (PEMA) oder Polypropylmethacrylat. Weiterhin können Polyvinylacetat, Polycyclohexylmethacrylate oder Polyphenylmethacrylat eingesetzt werden. Besonders bevorzugt wird Polymethylmethacrylat eingesetzt.

Auch Copolymere oder Polymermischungen aus beliebigen Anteilen der vorstehend genannten Polymere untereinander oder mit einer oder mehrerer weiterer Polymerkomponenten, beispielsweise Polystyrol, Polyacrylnitril oder Polyamiden können eingesetzt werden. Bevorzugt beträgt der Anteil der Monomere, die ein Strukturelement (A) aufweisen an solchen Mischpolymeren mindestens 20 %, besonders bevorzugt mindestens 40 % und ganz besonders bevorzugt mindestens 60 %.

Zur Modifikation solcher Oberflächen wird die Oberfläche mit den erfindungsgemäßen Irihibitorkonjugaten, z.B. in Form einer Lösung, in Kontakt gebracht. Dabei können auch Gemische unterschiedlicher Inhibitorkonjugate der vorliegenden Erfindung eingesetzt werden.

### Experimentelle Methoden:

### Abkürzungen.

ACN = Acetonitril
Amba =Amidinobenzylamid
Cha = Cyclohexylalanin
CKIBE = Chlorkohlensäureisobutylester
DIEA = Diisopropylethylamin
DMF = Dimethylformanlid
EE = Essigsäureethylester
AcOH = Essigsäure
HBTU = 2-(1H-Benzotriazole-1-yl)-1,1,3,3-Tetramethyluronium Hexafluorophosphat
4-MeO-bs = 4-Methoxy-benzensulfonyl
4-MeO-3-Cl-bs = 4-Methoxy-3-Chlor-benzensulfonyl
4-MeO-3-Me-bs = 4-Methoxy-3-Methyl-benzensulfonyl
Mtr = 4-Methoxy-2,3,6-trimethyl-benzensulfonyl
NMM = N-Methyl-Morpholin
OSu = Succinimidester
OtBu = tert.Butylester
PyBOP = Benzotriazole-1-yl-oxy-tris-pyrrolidino-phosphonium hexafluorophosphat
TFA = Trifluoressigsäure
THF = Tetrahydrofuran

Analytische HPLC: Shimadzu LC-10A System, Säule: Vydac C₁₈, 5 µm (250 x 4 mm) Lösungsmittel A: 0,1 % TFA in Wasser, B: 0,1 % TFA in ACN, Gradient: 10 % B bis 60 % B in 50 min, 1 ml/min Fluß, Detektion bei 220 oder 215 nm.

Präparative HPLC: Shimadzu LC-8A System, Säule: Vydac C₁₈, 10 µm (250 x 22 mm) Lösungsmittel A: 0,1 % TFA in Wasser, B: 0,1 % TFA in ACN, Gradient: 20 % B bis 65 % B in 120 min, 10 ml/min Fluß, Detektion bei 220 nm.

### Massenspektroskopie:

Die Massenspektren wurden auf einem Kompact Probe der Firma Kratos (Manchester, England) mit einem Flugzeitmessungsdetektor und α-Cyano-Hydroxyzimtsäure als Matrix gemessen.

### Bestimmung der kinetischen Konstanten

Zur Bestimmung der Thrombinhemmwirkung durch alle reversibel bindenden Inhibitoren und Inhibitor-Konjugate mit Hemmkonstanten ≥ 1 nM wurden 175 µl Tris-Puffer (0,05 mM, 0,1 M NaCI, pH 7,8; enthält den Inhibitor) und 50 µl Substrat (d-Phe-Gly-ArgpNA in H₂O, Konzentrationen im Messansatz 360, 120 und 60 µM) bei Raumtemperatur gemischt und die Reaktion durch Zugabe von 50 µl humanes' α-Thrombin (Kordia, finale Thrombinkonzentration im Messansatz 0,16 nM) gestartet. Über einen Zeitraum von 5 min wurde die Änderung der Absorption bei 405 nm mittels eines Microplate Readers (Labsystems iEMS Reader MF) bestimmt. Nach Berechnung der Anstiege (Reaktionsgeschwindigkeiten) wurden die Kᵢ-Werte nach Dixon (Biochem. J. 55, 170-171, 1953) durch lineare Regression mittels eines Computerprogramms ermittelt. Die Kᵢ-Werte sind das Mittel aus mindestens drei Bestimmungen.
Auf analoge Weise wurden die Kᵢ-Werte für Trypsin bestimmt.
Zur Bestimmung der Thrombinhemmwirkung durch alle reversibel bindenden Inhibitoren und Inhibitor-Konjugate mit Hemmkonstanten kleiner 1 nM wurde ein Fluoreszenzspektrometer (LS50B der Firma Perkin Elmer) verwendet. Der Messansatz besteht aus 880 µl Tris-Puffer (0,05 mM, 0,1 M NaCI, pH 7,8; enthält den Inhibitor, die Inhibitorkonzentration im Messansatz ist größer 200 pM) und 100 µl Substrat (Tos-Gly-Pro-Arg-AMC, Konzentrationen im Messansatz 5-30 µM). Die Messung wird durch Zugabe von 20 µl humanes α-Thrombin (Kordia, finale Thrombinkonzentration im Messansatz 21 pM) gestartet. Über einen Zeitraum von 7 min wurde die Zunahme der Fluoreszenzintensität verfolgt (λ_{Ex} 365 nm, λ_{Em} 455 nm). Nach Berechnung der Anstiege (Reaktionsgeschwindigkeiten) wurden die Kᵢ-Werte nach Dixon (Biochem. J. 55, 170-171, 1953) durch lineare Regression mittels eines Computerprogramms ermittelt. Die Kᵢ-Werte sind das Mittel aus mindestens drei Bestimmungen.
Wurde für die Inhibitoren ein slow-binding beobachtet, erfolgte die Auswertung analog einer früher beschriebenen Prozedur (Steinmetzer et al., Potent bivalent thrombin inhibitors: Replacement of the scissile peptide bond at P1-P1' with arginyl ketomethylene isosteres. J. Med. Chem. (1999) 42, 3109-3115).
Für die Optimierung der Inhibitor-Konjugate, insbesondere aus den nachfolgenden Beispielen 1 und 2, wurde nach geeigneten Resten R₁ gesucht, die eine möglichst starke Thrombinhemmung ermöglichen. Um die Selektivität der Verbindungen zu untersuchen wurden auch die Hemmkonstanten für Trypsin bestimmt Je größer der Quotient [Ki_{Trypsin}/Ki_{Thrombin}], desto selektiver ist die Verbindung als Thrombininhibitor. Um die Synthese zu erleichtern, wurde diese Optmierung zunächst ohne Kopplung mit einem Polyalkylenglykolrest an Inhibitorrnolekülen der Formel (II), wie in Tabelle I aufgeführt, vorgenommen. Diese Verbindungen, die selbst als Thrombininhibitoren aktiv sind, können als synthetische Zwischenstufen für die Inhibitorkonjugate der Formel (I) verwendet werden. Als zentraler Baustein wurde konstant ein Glycyl-Prolyl-Dipeptid in die Inhibitoren eingebaut.

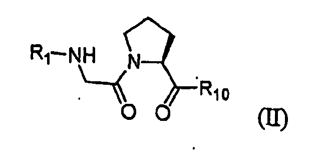

Die Ergebnisse der Selektivitätsbestimmung sind für beispielhafte Verbindungen in Tabelle I gezeigt; wobei R₁₀ für die Verbindungen der Tabelle die Struktur 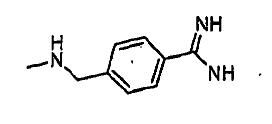 einnimmt.

**Tabelle 1:**

| Nr. | R₁ | Kᵢ Thrombin (nM) | Kᵢ Trypsin (nM) | Ki Trypsin/ Ki Thrombin |
|---|---|---|---|---|
| 1a | 4-Methoxy-3-Chlor-bs | 0,44 | 20 | 45 |
| 1b | 4-Methoxy-3-Methyl-bs | 0,64 | 13 | 20 |
| 1c | 4-Methoxy-bs* | 0,97 | 22,7 | 23,4 |
| 2 | Mtr** | 1,3 | 15,7 | 12,1 |
| 3 | 3-Methoxy-bs | 1,9 | 3 | 1,6 |
| 4a | 3,4-Dichlor-bs | 2,5 | 5,8 | 2,3 |
| 4b | . 3-Methyl-bs | 2,8 | 8 | 2,9 |
| 5 | 3-Chlor,4-Methyl-bs | 3,5 | 6,2 | 1,8 |
| 6 | Benzylsulfonyl | 3,6 | 7,4 | 2,1 |
| 7 | 3-Chlor-bs | 3,8 | 3,7 | 0,97 |
| 8 | 3,4-DiMethoxy-bs | 4,7 | 19,6 | 4,2 |
| 9 | 2,4,5-TriChlor-bs | 5,9 | 19,5 | 3,3 |
| 10 | 1-Naphthylsulfonyl | 7,8 | 3,9 | 0,5 |
| 11 | 3,5-DiChlor-bs | 8,5 | 3,4 | 0,4 |
| 12 | 2-Naphthylsulfonyl | 8,5 | 58 | 6,8 |
| 13 | 4-Chlor-bs | 10,0 | 27,3 | 2,7 |
| 14 | 2,4-DiChlor, 5-Methyl-bs | 10,5 | 11,8 | 1,1 |
| 15 | Bs | 10,8 | 10,4 | 0,96 |
| 16 | PentaMethyl-bs | 12,7 | 27,8 | 2,2 |
| 17 | 2,3-DiChlor-bs | 12,7 | 11,1 | 0,87 |
| 18 | 2,3,5,6-TetraMetbyl-bs | 13,4 | 34,0 | 2,5 |
| 19 | 2,4,6-TriIsopropyl-bs | 13,5 | 9,1 | 0,67 |
| 20 | Cyclohexylmethylsulfonyl | 13,6 | 3,1 | 0,22 |
| 21 | 2,4-DiChlor-bs | 15,1 | 17,5 | 1,2 |
| 22 | 4-Methyl-bs | 16,1 | 14,2 | 0,88 |
| 23 | 2,3,4-TriChlor-bs | 16,4 | 11,7 | 0,71 |
| 24 | 2,4,6-TriMethyl-bs | 16,6 | 13,4 | 0,80 |
| 25 | 2,4-DiChlor, 6-Methyl-bs | 16,9 | 19 . | 1,1 |
| 26 | 4-Ethyl-bs | 19,1 | 50,2 | 2,6 |
| 27 | 2-Chlor-bs | 19,3 | 19,1 | 0,98 |
| 28 | Pbf*** | 30,3 | 37,1 | 1,2 |
| 29 | 4-tert.Butyl-bs | 32,2 | 10,4 | 0,32 |
| 30 | 3-Nitro-bs | 38,0 | 16,1 | 0,42 |
| 31 | Phenylethylsutfonyt | 41,9 | 16,5 | 0,39 |
| 32 | trans-Styrensulfonyl | 44,3 | 2,1 | 0,05 |
| 33a | 3-Cyano-bs | 48 | 22,5 | 0,47 |
| 33b | 4-Ethoxy-bs | 66 | 28 | 0,42 |
| 34 | 4-Cyano-bs | 76 | 24,9 | 0,33 |
| 35 | 4-Nitro-bs | 115 | 12,6 | 0,11 |

| | | | | |
|---|---|---|---|---|
| *bs = Benzensulfonyl; **Mtr = 2,3,6-Trimethyl-4-methoxy-bs, ***Pbf= 2,2,4,6,7-Pentamethyldihydrobenzofuran-5-Sulfonyl | | | | |

Als besonders geeignete Thrombininhibitoren mit guter Trypsinselektivität erwiesen sich die Verbindungen der Struktur (II), bei denen R₁ einen 4-Methoxy-3-Chlor-benzensulfonyl-, 4-Methoxy-3-Methyl-benzensulfonyl-, 4-Methoxy-benzensulfonyl- oder 4-Methoxy-2,3,6-trimethyl-phenylsulfonylrest und R₁₀ einen Rest 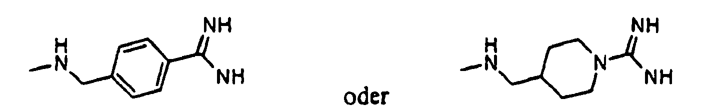 darstellt.

In Folge dieser Arbeiten wurde das Glycin durch trifunktionelle Aminosäuren ausgetauscht. Selektivitätswerte für die entstehenden Verbindungen der Struktur (III), die analog zu den in Tabelle 1 angegebenen Werten erhalten wurden, sind in Tabelle II aufgeführt. Dabei sind in Spalte 3 diejenigen Säurereste und Aminosäurereste aufgeführt, die von R₉ zusammen mit der Aminogruppe am benachbarten C-Atom und der Carbonylgruppe gebildet werden, die mit dem Stickstoff des Heterocyclus eine Amidbindung bildet Auch für die Verbindungen der Tabelle II ist R₁₀

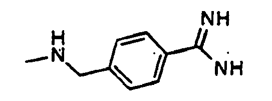

Verbindungen der Struktur (III), die selbst als Thrombininhibitoren aktiv sind, sind als synthetische Zwischenstufen einsetzbar.

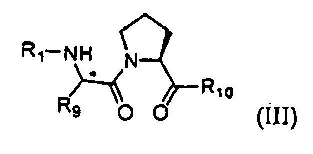

Besonders geeignet sind Verbindungen der Struktur III, für die
R₁ einen 4-Methoxy-3-Chlor-benzensulfonyl-, 4-Methoxy-3-Methyl-benzensulfonyl-, 4-Methoxy-phenylsulfonyl- oder einen 4-Methoxy-2,3,6-trimethylphenylsulfonylrest darstellt;
R₉ einen der Reste -(CH₂)ⱼ-C(O)OR₁₁, -(CH₂)ⱼ -R₁₂, oder -(CH₂)ⱼ -C(O)NH-R₁₁
darstellt, wobei R₁₁ ein Wasserstoffatom, oder ein C1-C6 Alkylrest, C7-C13 Aralkylrest oder ein C6 bis C12 Arylrest ist der gegebenenfalls substituiert sein kann, R12 eine der Gruppen NH₂, OH oder SH ist, und j 1, 2, 3 oder 4 ist,
R₁₀ 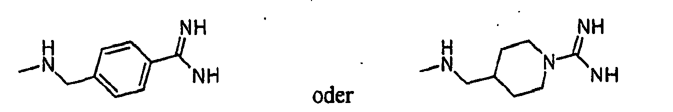 ist und
am mit * gekennzeichneten C-Atom eine L-Konfiguration vorliegt.

Geeignete Substituenten des Restes R₁₁ sind Halogenatome wie z.B. Chlor oder Brom, lineare oder verzweigte Alkylgruppen, Carboxylgruppen, Cyanogruppen, Carboxyalkylgruppen oder Aminoalkylgruppen, die im Falle des Aralkylrestes am Aromaten, aber auch am Alkylteil vorliegen können.

Beispielhafte Verbindungen der Formel (III) sind in Tabelle 2 zusammen mit ihren Selektivitätswerten aufgeführt.

**Tabelle 2:**

| Nr. | R₁ | -NH-CHR₉-CO- | Kᵢ Thrombin (nM) | Kᵢ Trypsin (nM) | Ki Trypsin/ Ki Thrombin |
|---|---|---|---|---|---|
| 36 | 2-Naphthylsulphonyl | Asp(OBzl) | 1,3 | 17,6 | 13,6 |
| 37 | 2-Naphthylsulphonyl | Diaminopropionsäure | 2,4 | 10,3 | 4,3 |
| 38 | 2-Naphthylsulphonyl | Ser | 2,54 | 27,6 | 10,9 |
| 39 | 2-Naphthylsulphonyl | Asn | 3,4 | 37,4 | 11 |
| 40 | 2-Naphthylsulphonyl | Gln | 3,8 | 16,1 | 4,2 |
| 41 | 2-Naphthylsulphonyl | Thr | 5,24 | 54,5 | 10,4 |
| 12 | 2-Naphthylsulphonyl | Gly | 8,5 | 58 | 6,8 |
| 42 | 2-Naphthylsulphonyl | Asp | 24,1 | 69 | 2,9 |
| 43 | 2-Naphthylsulphonyl | D-Asn | 52,5 | 22,4 | 0,42 |
| 44 | 4-Methoxy-bs | Asn(4-Cyano-Benzyl) | 0,045 | 41 | 911 |
| 45 | 4-Methoxy-bs | Asn(4-Aminomethyl-Benzyl) | 0,061 | 39 | 639 |
| 46 | 4-Methoxy-bs | Asn(3-Cyano-Benzyl) | 0,061 | 71 | 1163 |
| 47 | 4-Methoxy-bs | Asn(Benzyl) | 0,065 | 39 | 600 |
| 48 | 4-Methoxy-bs | Asn(3-Aminomethyl-Benzyl) | 0,08 | 41 | 512 |
| 49 | 4-Methoxy-bs | Asp(OBzl) | 0,21 | 38 | 181 |
| 50 | 4-Methoxy-bs | Asn | 0,36 | 53 | 147 |
| 51 | 4-Methoxy-bs | Ser | 0,255 | 17 | 67 |
| 52 | 4-Methoxy-bs | Asp(OMe) | 0,68 | 21,4 | 31 |
| 53 | 4-Methoxy-bs | Lys | 0,73 | 82 | 112 |
| 1c | 4-Methoxy-bs | Gly | 0,97 | 22,7 | 23,4 |
| 55 | 4-Methoxy-bs | Asp | 1,6 | 77 | 48 |

### Beispiele

### Beispiel 1

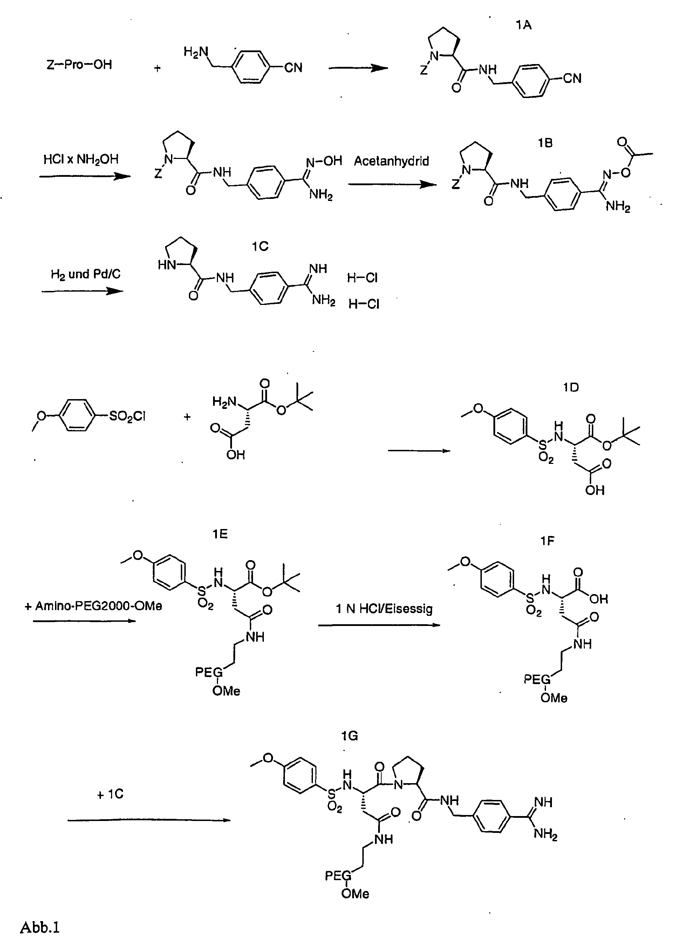

Herstellung von 4-Methoxy-benzensulfonyl-Asn(PEG2000-OMe)-Pro-4-Amidinobenzylamid (Die Synthese der Verbindung ist in der nachstehenden Abbildung 1 weiter veranschaulicht):
A) Z-Pro-4-(Cyano)Benzylamid
   7,27 g (29,16 mmol) Z-Pro-OH und 3,2 ml (29,16 mmol) NMM wurden in 200 ml THF unter Rühren gelöst. Der Ansatz wurde danach auf - 15 °C gekühlt und mit 3,79 ml (29,16 mmol) CKIBE versetzt. Nach ca. 10 min bei -15 °C wurden 4,24 g (32 mmol) 4-(Cyano)Benzylamin hinzugefügt und eine weitere Stunde bei -15°C und weitere 12 Stunden bei Raumtemperatur gerührt. Das Lösungsmittel wurde im Vakuum entfernt und der Rückstand in 500 ml EE gelöst und 3 x mit 5 % KHSO₄-Lösung, 1 x mit NaCl-gesättigter Lösung, 3 x mit NaHCO₃-gesättigter Lösung und 3 x mit NaCl-gesättigter Lösung gewaschen, mit Na₂SO₄ getrocknet und im Vakuum eingeengt. Ausbeute: 9,9 g (27,2 mmol) Öl, 93 %, HPLC: 31,67 min
B) Z-Pro-4(Acetyl-oxamidino)Benzylamid
   9,5 g (26,1 mmol) Z-Pro-4-(Cyano)Benzylamid wurden in 300 ml Methanol gelöst und mit 3,1 g (45 mmol) Hydroxylamin Hydrochlorid und 7,83 ml (45 mmol) DIEA unter Rühren versetzt. Der Ansatz wurde 4 h unter Rückfluss gekocht und über Nacht bei Raumtemperatur stehen gelassen. Das ausgefallene Zwischenprodukt (HPLC 18,18 min) wurde abgesaugt, im Vakuum getrocknet, bei Raumtemperatur in 200 ml AcOH gelöst und in 3 Portionen mit 75 mmol Acetanhydrid versetzt. Nach 30 Minuten wurde das Lösungsmittel und überschüssiges Acetanhydrid im Vakuum entfernt und der verbleibende Rückstand in EE gelöst. Es wurde 3 x mit 5 % KHSO₄-Lösung und 3 x mit NaCl-gesättigter Lösung gewaschen, dabei fällt bereits das Produkt als Feststoff aus, das abgesaugt, mit Wasser auf der Fritte gewaschen und im Vakuum getrocknet wurde. Ausbeute: 6,4 g (14,6 mmol) 56 %; weiße Kristalle, HPLC: 24,37 min
C) H-Pro-4(Amidino)Benzylamid x 2 HCl
   6 g (13,6 mmol) Z-Pro-4(Acetyl-oxamidino)Benzylamid werden in 200 ml 90%iger AcOH unter Rühren gelöst und unter Schutzgas mit 0,5 g 10 %igem Palladium auf Aktivkohle versetzt. Dann wird mittels Wasserstoff 12 h hydriert und der Katalysator abgefiltert. Das Filtrat wird im Vakuum eingeengt, der Rückstand in 100 ml 0,5 N HCl gelöst und danach wieder im Vakuum eingeengt und getrocknet.
   Ausbeute: 3,3 g (10,3 mmol) 76 % Feststoff, HPLC: 11,3 min (Start bei 0 % B)
D) 4-MeO-benzensulfonyl-Asp-OtBu
   10 g (52,8 mmol) H-Asp-OtBu (Novabiochem) wurden in 250 ml H₂0 und 150 ml ACN unter Rühren bei Raumtemperatur suspendiert und mit 13 ml (75 mmol) DIEA versetzt. Der Ansatz wurde im Eisbad gekühlt. Dann wurden innerhalb einer Stunde 11,7 g (55 mmol) 4-Methoxy-benzensulfonylchlorid, gelöst in 100 ml ACN, zugetropft. Der pH-Wert wurde während dieses Zeitraums kontrolliert und durch zusätzliche DIEA-Zugabe auf 8-9 eingestellt. Danach wurde der Ansatz weitere 6 Stunden bei Raumtemperatur und bei einem pH-Wert von 8-9 gerührt. Dann wurde das Lösungsmittel im Vakuum entfernt und der verbleibende Rückstand in 300 ml Wasser bei einem pH-Wert von ca. 9 gelöst. Die wässrige Phase wurde 2 x mit Ether extrahiert und danach durch Zusatz von 5 %iger KHSO₄-Lösung auf einen pH-Wert von ca. 3 eingestellt. Die saure, wässrige Phase wurde anschließend 3 x mit EE extrahiert, die vereinigte EE-Phase wurde danach noch 2 x mit 5 %iger KHSO₄-Lösung und 3 x mit gesättigter NaCl-Lösung gewaschen und mit Na₂SO₄ getrocknet. Danach wurde der EE im Vakuum entfernt, das verbleibende Öl wurde in heißem EE gelöst und mit Hexan überschichtet. Das Produkt kristallisierte bei ca. 4 °C.
   Ausbeute: 13,6 g, HPLC bei 28,9 min
E) 4-MeO-bs-Asn(PEG₂₀₀₀-OMe)-OtBu
   5 g (ca. 2,5 mmol) Amino-PEG₂₀₀₀-Monomethylether (Rapp Polymere, Tübingen) und 2,7 g (7,5 mmol) 4-MeO-bs-Asp-OtBu wurden in 200 ml DMF und 50 ml ACN bei Raumtemperatur gelöst und danach im Eisbad abgefühlt. Dann wurden 2,84 g (7,5 mmol) HBTU und 3,48 ml (20 mmol) DIEA hinzugegeben und der Ansatz für weitere 30 min unter Eiskühlung und anschließend weitere 5 h bei Raumtemperatur gerührt Das Lösungsmittel wurde im Vakuum eingeengt und der verbleibende Rückstand in wenig heißem Methanol gelöst und mit einem großen Überschuß an Ether versetzt. Der Ansatz wurde für eine Stunde ins Eisbad gestellt, danach wurde das ausgefallene Produkt abgesaugt und mit Ether gewaschen und getrocknet Die Festsubstanz wird wieder in wenig, heißem Methanol gelöst und mit einem großen Überschuß an Isopropanol versetzt. Beim Stehen im Eisbad fällt das Produkt aus, es wird abgesaugt und auf der Fritte mit Isopropanol gewaschen und danach im Vakuum getrocknet. Die Verbindung wird getrocknet und in dieser Form für die weitere Synthese verwendet.
   Ausbeute: 5,5 g, HPLC 35,1 min
F) 4-MeO-bs-Asn(PEG₂₀₀₀-OMe)-OH
   5 g 4-MeO-bs-Asn(PEG₂₀₀₀-OMe)-OtBu wurden bei Raumtemperatur in 150 ml 1 N HCl in Essigsäure gelöst, der Ansatz wurde 4 h bei Raumtemperatur stehen gelassen und danach das Lösungsmittel im Vakuum entfernt. Der Rückstand wird in Toluol gelöst und danach das Lösungsmittel wieder im Vakuum abgedampft, um Säurespuren zu entfernen. Dieser Schritt wird noch 2 x wiederholt, der Rückstand in wenig heißem Methanol gelöst und das Produkt mit Ether in der Kälte gefällt. Nach Absaugen und Waschen mit Ether wird das Produkt im Vakuum getrocknet und in dieser Form für den nächsten Syntheseschritt eingesetzt.
   Ausbeute: 4,7 g, HPLC 30,5 min
G) 4-Methoxy-benzensulfonyl-Asn(PEG₂₀₀₀-CMe)-Pro-4-Amidinobenzylamid
   2 g (ca. 0,9 mmol) 4-MeO-bs-Asn(PEG₂₀₀₀-OMe)-OH und 0,57 g (1,8 mmol) des unter C) beschriebenen H-Pro-4-Amidinobenzylamids x 2 HCl werden in 50 ml DMF unter Rührem gelöst. Nach Kühlung mit einem Eisbad werden 0,94 g (1,8 mmol) PyBOP und 0,93 ml (5,4 mmol) DIEA hinzugefügt. Der Ansatz wurde 30 Minuten unter Eiskühlung und weitere 4 h bei Raumtemperatur gerührt, das Lösungsmittel im Vakuum eingeengt und der verbleibende Rückstand an Biogel P2 (Firma Biorad) mit 2 %iger Essigsäure als Laufmittel getrennt. Die Produkt-enthaltenden Fraktionen wurden vereinigt, im Vakuum eingeengt und aus 80 %igem tert. Butanol lyophilisiert. In einem zweiten Schritt wurde anschließend das Produkt durch Ionenaustauschchromotographie an Fractogel EMD COO⁻ oder Source 30Smit einem Ammoniumacetatgradienten getrennt und die vereinten Fraktionen 3 x nacheinander lyophilisiert.
   HPLC: 30,66 min

### Beispiel 2:

In analoger Weise zu Beispiel 1 wurden die folgenden Verbindungen synthetisiert, wobei für die Synthese Amino-PEG-Verbindungen mit verschiedenen Molekulargewichten verwendet wurden.
4-MeO-bs-Asn(PEG₅₀₀₀-OMe)-Pro-4-Amidinobenzylamid
4-MeO-bs-Asn(PEG₁₀₀₀₀-OMe)-Pro-4-Amidinobenzylamid
4-MeO-bs-Asn(PEG₂₀₀₀₀-OMe)-Pro-4-Amidinobenzylamid
Mtr-Asn(PEG₂₀₀₀-OMe)-Pro-4-Amidinobenzylamid
Mtr-Asn(PEG₅₀₀₀-O-OMe)-Pro-4-Amidinobenzylamid
Mtr-Asn(PEG₁₀₀₀₀-OMe)-Pro-4-Amidinobenzylamid
Mtr-Asn(PEG₂₀₀₀₀-OMe)-Pro-4-Amidinobenzylamid

Durch Verwendung eines bifunktionalisierten Amino-PEG wurden die beiden folgenden Verbindungen hergestellt, wobei die Verbindungslinie in dieser und den folgenden Formeln die Verknüpfung der beiden Inhibitoreinheiten mittels des angegeben bifunktionellen Polyalkylenglykols darstellt.

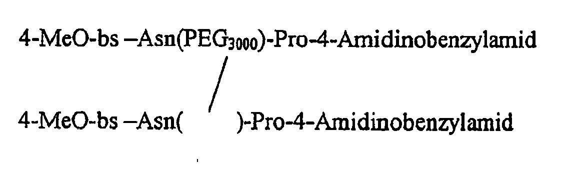

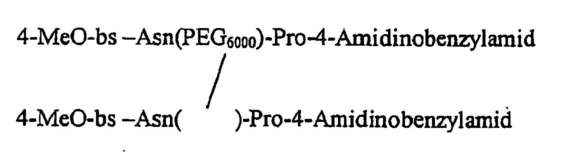

Durch Verwendung von Aminomethyl-Crown-Ethern wurden die folgenden Verbindungen hergestellt:
4-MeO-bs-Asn(CH₂-Crown5)-Pro-4-Arnidinobenzylamid
4-MeO-bs-Asn(CH₂-Crown6)-Pro-4-Amidinobenzylamid

### Beispiel 3

### Herstellung von D-N(CH₂-CO-NH-PEG₂₀₀₀-OMe)Cha-Pro-4-Amidinobenzylamid

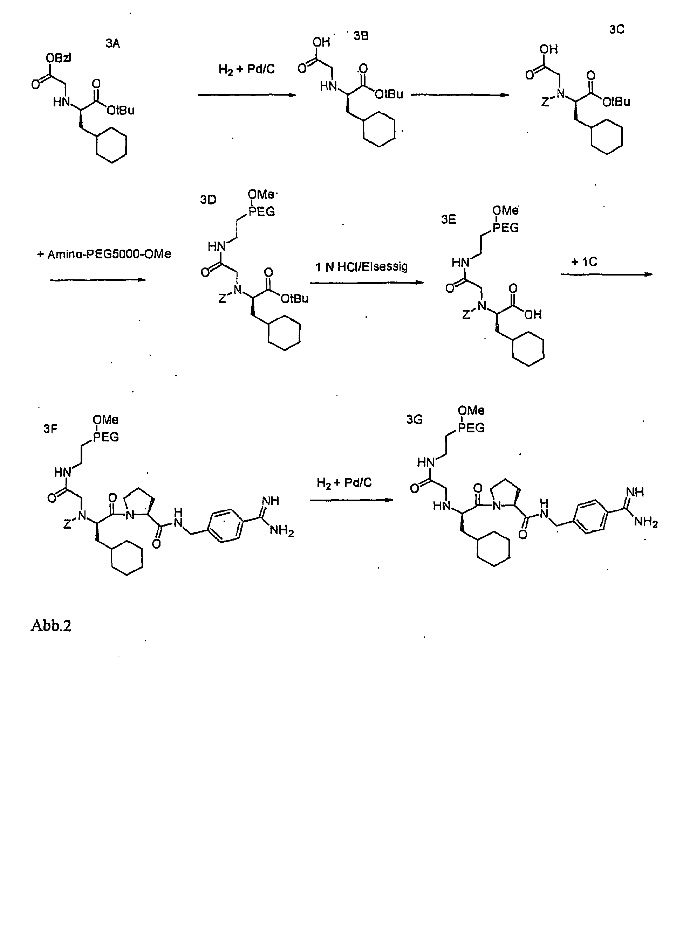

(Die Synthese der Zielverbindung ist in der nachfolgenden Abbildung 2 weiter veranschaulicht):
A) D-N(Benzyloxycarbonylmethyl)Cha-OtBu
   Der Ausgangsstoff D-N(Benzyloxycarbonylmethyl)Cha-OtBu wurde entsprechend der beschriebenen Literaturmethode hergestellt (Preville et al., Bioorganic & Medicinal Chemistry Letters 7, 1563-1566 (1997).
B) D-N(Carboxymethyl)Cha-OtBu x Acetat
   3,75 g (10 mmol) D-N(Benzyloxycarbonylmethyl)Cha-OtBu wurden in einer Mischung aus 100 ml Methanol, 5 ml Essigsäure und 5 ml H₂O bei Raumtemperatur und Normaldruck 3 h mit 0,3 g 10 % Pd/C als Katalysator wie üblich hydriert. Der Katalysator wurde abfiltriert, und das Filtrat unter Zusatz von Toluen 3Mal im Vakuum eingeengt. Die verbleibende Substanz wird in 15 ml Essigsäure gelöst und unter Zusatz von 200 ml Diethylether gefällt (weißer Feststoff).
C)Z-D-N(Carboxymethyl)Cha-OtBu x Cyclohexylamin
   2 g (5,8 mmol) D-N(Carboxymethyl)Cha-OtBu x Acetat wurden in 75 ml Wasser, 15 ml 1 N NaOH und 75 ml Dioxan unter starkem Rühren suspendiert und im Eisbad gekühlt. Danach wurden 7 mmol Z-Cl, gelöst in 30 ml Dioxan, im Verlauf von 30 Minuten zugetropft, noch 30 Minuten unter Eiskühlung und weitere 12 h bei Raumtemperatur gerührt. Der pH-Wert wurde durch Zugabe von 1 N NaOH auf ca. 9-10 mehrmals nachgestellt. Danach wurde das Lösungsmittel im Vakuum entfernt und der Rückstand in EE und Wasser aufgenommen und 3 x mit 5 %iger KHSO₄-Lösung und 3 x mit NaCl-gesättigter Lösung gewaschen, mit Na₂SO₄ getrocknet und im Vakuum eingeengt. Der ölige Rückstand wurde in Ether gelöst und als Cyclohexylamin-Salz kristallisiert, abgesaugt und im Vakuum getrocknet.
D) Z-D-N(CH₂-CO-NH-PEG₅₀₀₀-OMe)Cha-OtBu
   0,62 g (1,2 mmol) Z-D-N(Carboxymethyl)Cha-OtBu x Cyclohexylamin wurden in 200 ml EE suspendiert und 3 x mit 50 ml 5 % KHSO₄-Lösung und 3 x mit NaCl-gesättigter Lösung gewaschen, mit Na₂SO₄ getrocknet und im Vakuum eingeengt. Der ölige Rückstand und 2 g (0,4 mmol) Amino-PEG5000-OMe wurden in 100 ml DMF unter Rühren gelöst und im Eisbad abgekühlt. Dazu wurden 0,62 g (1,2 mmol) PyBOP und 3 mmol DIEA gegeben, weitere 30 Minuten unter Eiskühlung und danach 12 h bei Raumtemperatur gerührt. Das Lösungsmittel wurde im Vakuum verdampft, der Rückstand in wenig, heißem Methanol gelöst und in der Kälte mit Isopropanol gefällt, abgesaugt und im Vakuum getrocknet. Die Festsubstanz wurde wieder in wenig, heißem Methanol gelöst und in der Kälte mit Ether gefällt, abgesaugt und im Vakuum getrocknet (Feststoff).
   Ausbeute: 1,9 g
E) Z-D-N(CH₂-CO-NH-PEG₅₀₀₀-OMe)Cha-OH
   Der tert.Butylester wurde wie im Beispiel 1F beschrieben abgespalten.
   Ausbeute: 1,7 g (Feststoff)
F) Z-D-N(CH₂-CO-NH-PEG₅₀₀₀-OMe)Cha-Pro-4-(Amidino)Benzylamid
   Das unter Beispiel 1C hergestellte H-Pro-4(Amidino)Benzylamid x 2 HCl wurde entsprechend der Vorschrift aus Beispiel 1G an 1 g Z-D-N(CH₂-CO-NH-PEG₅₀₀₀-OMe)Cha-OH angekoppelt Das Produkt wurde nacheinander mit Isopropanol und Ether gefällt, abgesaugt und im Vakuum getrocknet (Rohprodukt: 0,9 g).
G) H-D-N(CH₂-CO-NH-PEG₅₀₀₀-OMe)Cha-Pro-4-(Amidino)Benzylamid
   0,9 g Z-D-N(CH₂-CO-NH-PEG₅₀₀₀-OMe)Cha-Pro-4-(Amidino)Benzylamid wurden wie in Beispiel 1C beschrieben 4 h hydriert, nach abfiltrieren des Katalysators, einengen des Lösungsmittels im Vakuum, wurde der Rückstand in wenig heißem Methanol gelöst und mit viel Ether gefällt und abgesaugt. Das gefällte Rohprodukt wurde an Biogel P2 mit 2 % AcOH als Laufmittel gereinigt. In einem zweiten Schritt wurde anschließend das Produkt durch Ionenaustauschchromotographie an Fractogel EMD COO⁻ oder Source 30S mit einem Ammoniumacetatgradienten getrennt und die vereinten Fraktionen 3 x nacheinander lyophilisiert.

### Beispiel 4

In analoger Weise zum Beispiel 3 wurden die folgenden Verbindungen synthetisiert, wobei für die Synthese Amino-PEG-Verbindungen init verschiedenen Molekulargewichten verwendet wurden.
H-D-N(CH₂-CO-NH-PEG₂₀₀₀-OMe)Cha-Pro-4-(Amidino)Benzylamid
H-D-N(CH₂-CO-NH-PEG₁₀₀₀₀-OMe)Cha-Pro-4-(Amidino)Benzylamid
H-D-N(CH₂-CO-NH-PEG₂₀₀₀₀-OMe)Cha-Pro-4-(Amidino)Benzylamid

Durch Verwendung eines bifunktionalisierten Amino-PEG wurden die folgenden Verbindungen hergestellt:

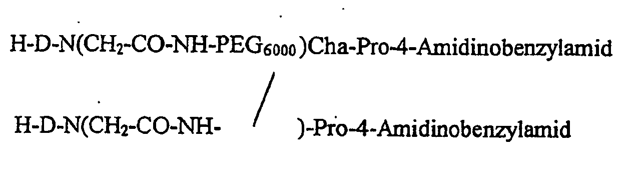

Durch Verwendung von Aminomethyl-Crown-Ethern wurden die folgenden Verbindungen hergestellt:
H-D-N(CH₂-CO-NH-CH₂-Crown5)-Pro-4-Amidinobenzylamid
H-D-N(CH₂-CO-NH-CH₂-Crown6)-Pro-4-Amidinobenzylamid

### Beispiel 5

### D-N(CH₂-CO-NH-PEG₅₀₀₀-OMe)Cha-Pro-4-(Amidomethyl)N(Amidino)Benzylamid

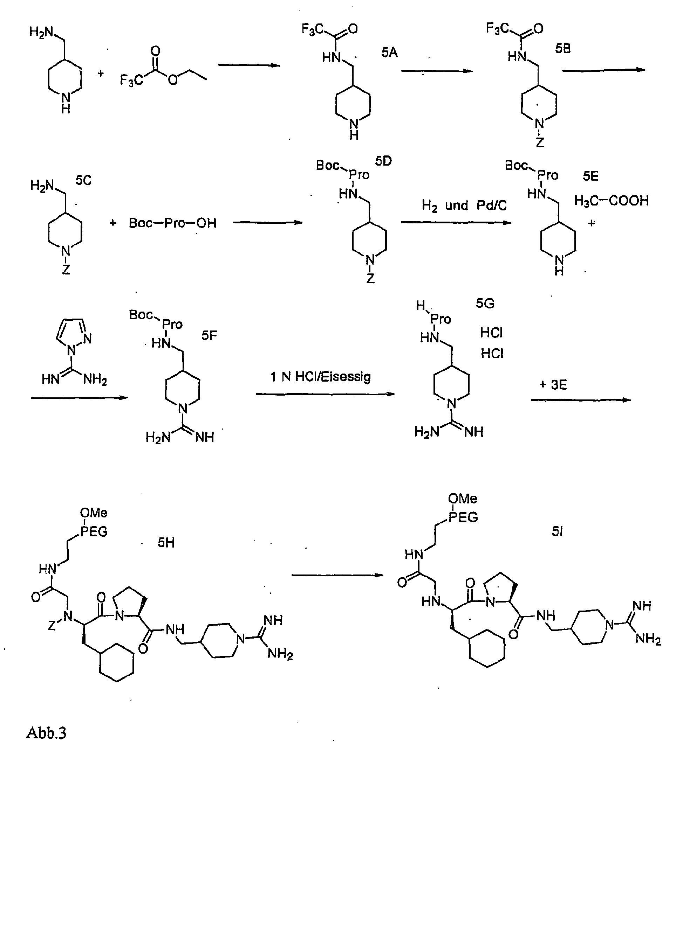

(Die Synthese der Zielverbindung ist in der nachfolgenden Abbildung 3 weiter veranschaulicht):
A) 4-(Trifluoroacetyl-Amidomethyl)-Piperidin
   166 mmol (18,95 g, 19.91 ml) 4-Aminomethylpiperidin werden in 100 ml DCM gelöst und unter magnetischen Rühren im Eisbad gekühlt. Über einen Zeitraum von einer. Stunde werden 182.6 mmol (25.94 g, 21.78 ml) Trifluoroessigsäureethylester zugetropft. Danach wird der Ansatz eine weitere Stunde im Eisbad gekühlt und danach noch 5 Stunden bei Raumtemperatur gerührt. Das während der Reaktion ausfallende Produkt wird abgesaugt.
   Ausbeute: 18.68 g = 53.55 %
   MS: berechnet, 210.17; gefunden 211,3 [M+H]⁺ (Maldi)
   DC: Rf n-Butanol/Eisessig/Wasser 4/1/1 = 0.51
B) 4-(Trifluoroacetyl-Amidomethyl)-N(Benzyloxycarbonyl)-Piperidin
   Zu einer Lösung von 18 g (85.6 mmol) 4-(Trifluoroacetyl-Amidomethyl)-Piperidin in 150 ml DMF werden 14.89 ml (85.6 mmol) DIEA hinzugegeben. Unter Rühren im Eisbad gibt man zu dieser Mischung 22.36 g (89.75 mmol) Z-Osu, das vorher in 75 ml DMF gelöst wurde. Der Ansatz wird 1 Stunde im Eisbad und danach 7 Stunden bei Raumtemperatur gerührt. Danach wird das Lösungsmittel im Vakuum eingeengt und der verbleibende Rest in Essigester aufgenommen und 3 x mit 5%iger KHSO₄-Lösung und 3 x mit gesättigter NaCl-Lösung gewaschen. Die EE-Phase wird über Na₂SO₄ getrocknet, und das Lösungsmittel eingeengt. Umkristallisation aus Essigester/Hexan (weiße Kristalle).
   Ausbeute: 28.15 g = 95.52 %
   MS: berechnet: 344.27; gefunden: 345,1
   DC: Rf n-Butanol/Eisessig(Wasser 4/1/1 = 0.83
   Benzen/Aceton/Eisessig 27/10/0.5 = 0.73
C) 4-(Aminomethyl)-N(Benzyloxycarbonyl)-Piperidin
   27.5 g (79.88 mmol) 4-(Trifluoroacetyl-Amidomethyl)-N(Benzyloxycarbonyl)-Piperidin werden in 150 ml Dioxan gelöst und mit 150 ml 1 n NaOH-Lösung versetzt. Der Ansatz wird 3 Stunden bei 40° C gerührt und danach das Lösungsmittel im Vakuum eingeengt. Der Rest wird in Wasser aufgenommen und 3 x mit DCM extrahiert. Die vereinigten DCM-Phasen werden über Na₂SO₄ getrocknet, danach wird das Lösungsmittel im Vakuum eingeengt.
   Ausbeute: 13.92 g Öl, 56.09 mmol = 70.2 %
   MS: berechnet, 248,13; gefunden, 249,3 [M+M]⁺
D) Boc-Pro-4-(Amidomethyl)-N(Benzyloxycarbonyl)-Piperidin
   0,86 g Boc-Pro-OH werden in 60 ml absolutem THF unter magnetischem Rühren gelöst und mit 0.44 ml (4 mmol) NMM versetzt. Der Ansatz wird auf -15° C gekühlt und dann mit 4 mmol (0.52 ml) CKIBE versetzt. Nach weiteren 10 Minuten Rühren bei -15° C werden 1.1 g (4.4 mmol) 4-(Aminomethyl)-N(Benzyloxycarbonyl)-Piperidin zugesetzt und der Ansatz eine weitere Stunde bei -15° C und weiter über Nacht bei Raumtemperatur gerührt Das Lösungsmittel wird im Vakuum eingeengt und der verbleibende Rest in Essigester aufgenommen und nacheinander 3 x mit 5% KHSO₄-Lösung, 1 x mit gesättigter NaCl-Lösung, 3 x mit gesättigter NaHCO₃-Lösung und nochmals 3 x mit gesättigter NaCl-Lösung gewaschen. Danach wird die Essigesterphase mit Na₂SO₄ getrocknet und eingeengt.
   Umkristallisation aus Essigester/Hexan.
   Ausbeute: 1.5 g, 3,3 mmol (82,5 %)
E) Boc-Pro-4-(Amidomethyl)Piperidin x Acetat 1 g (2,2 mmol) Boc-Pro-4-(Amidomethyl)-N(Benzyloxycarbonyl)-Piperidin werden in Analogie zum Beispiel 1C mit 150 mg Pd/C als Katalysator in Eisessig hydriert. Der Katalysator wird abfiltriert und das Lösungsmittel weitestgehend im Vakuum eingeengt, und das Produkt durch Zusatz mit Diethylether gefällt.
   Ausbeute: 0,6 g
F) Boc-Pro-4-(Amidomethyl)-N(Amidino)Piperidin x TFA
   0,5 g (1,34 mmol) Boc-Pro-4-(Amidomethyl)Piperidin x Acetat werden mit 0,4 g (2,7 mmol) Pyrazol-Carboxamidin-Hydrochlorid und 0,7 ml(4 mmol) DIEA in 4 ml DMF bei Raumtemperatur für 24 h gerührt Das DMF wird im Vakuum entfernt und der Rückstand mittels präparativer Reversed-Phase-HPLC von Salzen und nicht umgesetzten Pyrazol-Carboxamidin-Hydrochlorid getrennt.
   Ausbeute: 0,35 g
G) H-Pro-4-(Amidomethyl)-N(Amidino)Piperidin x 2 HCl
   0,3 g (0,64 mmol) Boc-Pro-4-(Amidomethyl)-N(Amidino)Piperidin x TFA werden mit 5 ml 1 N HCl/Eisessig übergossen und bei Raumtemperatur 1 Stunde stehen gelassen. Der Eisessig wird im Vakuum weitestgehend eingeengt und der Rückstand durch Zugabe von Diethylether gefällt. Das Produkt wird abgesaugt, mit weiterem Diethylether gewaschen und im Vakuum getrocknet.
   Ausbeute: 0,18 g
H) Z-D-N(CH₂-CO-NH-PEG₅₀₀₀-OMe)Cha-Pro-4-(Amidomethyl)-N(Amidino)Piperidin x HCl
   0,15 g (0,46 mmol) des unter Beispiel 5G hergestellten H-Pro-4-(Amidomethyl)-N(Amidino)Piperidin x 2 HCl wurde entsprechend der Vorschrift aus Beispiel 1G an 1 g Z-D-N(CH₂-CO-NH-PEG₅₀₀₀-0Me)Cha-OH mittels PyBop/DIEA angekoppelt. Das Produkt wurde nacheinander mit Isopropanol und Ether gefällt abgesaugt und im Vakuum getrocknet (Rohprodukt: 0,9 g).
I) D-N(CH₂-CO-NH-PEG₅₀₀₀-OMe)Cha-Pro-4-(Amidomethyl)-N(Amidino)Piperidin x 2 HCl
   0,8 g Z-D-N(CH₂-CO-NH-PEG₅₀₀₀-OMe)Cha-Pro-4-(Amidomethyl)-N(Amidino)Piperidin x HCl werden in 90 %iger Essigsäure analog der Vorschrift 1C hydriert. Das Produkt wird aus Methanol/Ether gefällt und über Biogel P2 und Ionenaustauschchromatographie (Fractogel EMD COO⁻ oder Source 30S ) gereinigt und lyophilisiert
   Ausbeute: 0,46 g

### Beispiel 6

In analoger Weise zum Beispiel 5 wurden die folgenden Verbindungen synthetisiert, wobei für die Synthese Amino-PEG-Verbindungen mit verschiedenen Molekulargewichten verwendet wurden.
H-D-N(CH₂-CO-NH-PEG₂₀₀₀-OMe)Cha-Pro-4-(Amidomethyl)-N(Amidino)Piperidin x 2 HCl
H-D-N(CH₂-CO-NH- PEG₁₀₀₀₀-OMe)Cha-Pro-4-(Amidomethyl)-N (Amidino)Piperidin x 2HCl
H-D-N(CH₂-CO-NH-PEG₂₀₀₀₀-OMe)Cha-Pro-4-(Amidomethyl)-N(Amidino)Piperidin x 2 HCl

Durch Verwendung eines bifunktionalisierten Amino-PEG wurden die folgenden Verbindungen hergestellt:

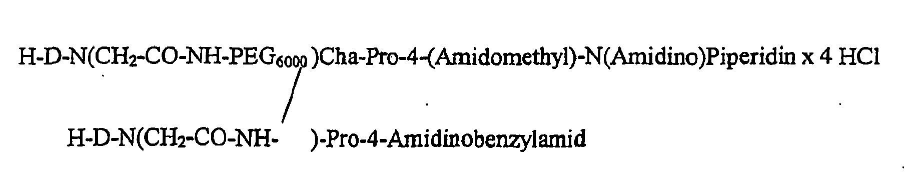

Durch Verwendung von Aminomethyl-Crown-Ethern wurden die folgenden Verbindungen hergestellt:
H-D-N(CH₂-CO-NH-CH₂-Crown5)-Pro-4-(Amidomethyl)-N(Amidino)Piperidin x 2 HCl
H-D-N(CH₂-CO-NH-CH₂-Crown6)-Pro-4-(Amidomethyl)-N(Amidino)Piperidin x 2 HCl

### Beispiel 7

### Herstellung von D-N(CH₂-CO-NH-PEG₂₀₀₀-OMe)Cha-Prg-Arg-CH₂Cl

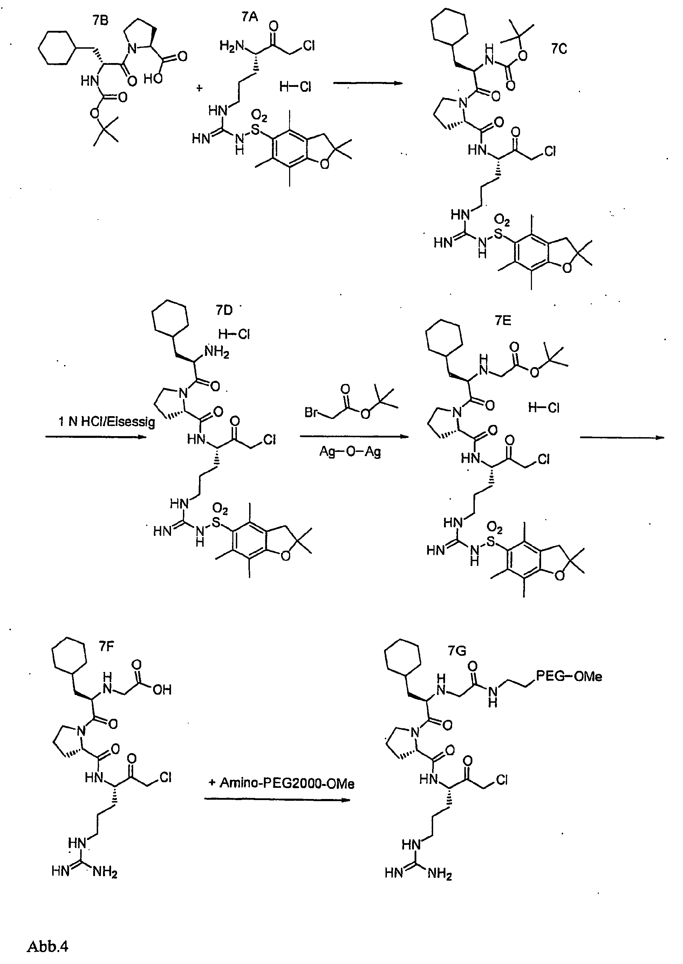

(Die Synthese der Zielverbindung ist in der nachfolgenden Abbildung 4 weiter veranschaulicht):
A) H-Arg(Pbf)-CH₂-Cl x HCl
   Boc-Arg(Pbf)-CH₂-Cl wurde analog der Literaturvorschrift für Boc-Arg(Z₂)-CH₂-Cl (Steinmetzer et al., 1999 J. Med. Chem. 42, 3109-3115) hergestellt. Durch versetzen von 5g Boc-Arg(Pbf)-CH₂-Cl mit 75 ml 1 N HCl in Eisessig wurde selektiv die Boc-Gruppe abgespalten, das Lösungsmittel weitestgehend im Vakuum entfernt und das Produkt durch Zusatz von kaltem Diethylether gefällt, abgesaugt und im Vakuum getrocknet Ausbeute: 3,7 g
B) Boc-d-Cha-Pro-OH.
   Die Verbindung wird entsprechend der in der Literatur beschriebenen Synthesestrategie hergestellt (Steinmetzer et al., 1999 J. Med. Chem. 42, 3109-3115).
C) Boc-d-Cha-Pro-Arg(Pbf)-CH₂-Cl
   Aus 3 g (8.14 mmol) Boc-d-Cha-Pro-OH, 0.9 ml (8.14 mmol) NMM und 1,06 ml (8.14 mmol) CKIBE in 100 ml THF wird bei -15 °C das gemischte Anhydrid gebildet. Nach 10 min werden 4,03 g (8.14 mmol) H-Arg(Pbf)-CH₂-Cl x HCl hinzugefügt und der Ansatz eine weitere Stunde bei -15 °C und über Nacht bei Raumtemperatur gerührt und wie unter Beispiel 1A beschrieben, aufgearbeitet
   Ausbeute: 4.6 g (5.7 mmol)
D) H-d-Cha-Pro-Arg(Pbf)-CH₂-Cl x HCl
   Die Boc-Gruppe wird analog der Vorschrift aus Beispiel 5G abgespalten, das Produkt mit Ether gefällt, abgesaugt und im Vakuum getrocknet.
   Ausbeute: 3.86 g (5.2 mmol)
E) H-d-N(CH₂-CO-OtBut)Cha-Pro-Arg(Pbt)-CH₂-Cl x HCl
   3 g (4 mmol) H-d-Cha-Pro-Arg(Pbf)-CH₂-Cl x HCl werden in 100 ml DMF gelöst und bei Raumtemperatur mit 0,74 ml (4.15 mmol) Bromessigsäure-tert.Butylester und 1,16 g (5 mmol) Silber(I)Oxid versetzt. Der Ansatz wird über Nacht gerührt und danach die ausgefallenen Silbersalze abzentrifugiert. Das DMF wird im Vakuum entfernt, der Rückstand in Essigester aufgenommen und 2 x mit NaCl-gesättigtem Wasser gewaschen, mit Na₂SO₄ getrocknet und eingeengt.
   Ausbeute: 2.7 g (3.1 mmol) Rohprodukt
F) H-d-N(CH₂-COOH)Cha-Pro-Arg-CH₂Cl x 2 Trifluoressigsäure
   2,5 g (2,87 mmol) des Rohproduktes an H-d-N(CH₂-CO-OtBut)Cha-Pro-Arg(Pbf)-CH₂-Cl x HCl werden bei Raumtemperatur mit 50 ml 90% Trifluoressigsäure in Wasser versetzt und 1,5 h bei Raumtemperatur gerührt. Das Lösungsmittel wird im Vakuum weitestgehend entfernt und der Rückstand mit kaltem Diethylether gefällt. Das Rohprodukt wird abgesaugt, mit Ether gewaschen, getrocknet und mit präparativer reversed-phase HPLC gereinigt.
G) H-d-N(CH₂-CONH-PEG₂₀₀₀-CMe)Cha-Pro-Arg-CH₂-Cl x 2 Trifluoressigsäure
   40 mg (0,054 mmol) HPLC gereinigtes H-d-N(CH₂-COOH)Cha-Pro-Arg-CH₂-Cl x 2 Trifluoressigsäure und 80,75 mg (0,04 mmol) Amino-PEG₂₀₀₀-OMe werden in 3 ml DMF gelöst und unter Eiskühlung mit 57 µl Propanphosphonsäureanhydrid, sowie 55 µl DIEA versetzt. Nach einer Stunde wird das Eisbad entfernt, und der Ansatz weitere 3 h bei Raumtemperatur gerührt. Das Lösungsmittel wird im Vakuum entfernt und der Rückstand mittel präparativer Umkehrphasenchromatographie gereinigt.
   Ausbeute: 45 mg

### Beispiel 8

### Bestimmung der kinetischen Konstanten

Bis auf die im Beispiel 6 beschriebene Verbindung sind sämtliche Inhibitoren reversibel bindende Thrombininhibitoren. Die bestimmten Kᵢ-Werte sind nur in geringem Maße von der Kettenlänge des PEG abhängig, daher ist für alle Verbindungstypen exemplarisch jeweils nur ein Inhibitor mit seinem Kᵢ-Wert angegeben, als Referenz werden die kinetischen Konstanten für die Verbindungen mit freier Carboxylgruppe, die keine PEG-Kette gebunden haben, angegeben.
*Kinetische Konstanten für den reversibel bindenden Inhibitor aus Beispiel 1*/*2*
4-Methoxy-benzensulfonyl-Asn(PEG2000-OMe)-Pro-4-Amidinobenzylamid
Ki_{Thrombin}: 0,53 nM; Ki_{Trypsin}: 92,53 nM; Ki_{Trypsin}/Ki_{Thrombin}: 174
Referenz: 4-Methoxy-benzensulfonyl-Asp-Pro-4-Amidinobenzylamid
Ki_{Thrombin}: 1,6 nM; Ki_{Trypsin}: 77 nM; Ki_{Trypsin}/Ki_{Thrombin}: 48

Bei den Inhibitoren dieses Typs führt die Einführung der PEG-Kette zu einer Verbesserung der Thrombinhemmung im Vergleich zu der gewählten Referenzverbindung und auch zu einer verbesserten Selektivität gegenüber Trypsin.
4-Methoxy-benzensulfonyl-Asn(4-[MeO-PEG₅₀₀₀-CH₂CH₂CO-NHCH₂]-Benzyl)-Pro-4-Amidinobenzylamid
   Ki _{Thrombin} 0,19 nM
Referenz: 4-Methoxy-benzensulfonyl-Asp-Pro-4-Amidinobenzylamid
   Ki_{Thrombin}: 1,6 nM;
4-Methoxy-benzensulfonyl-Lys(Propionyl-PEG₅₀₀₀-OMe)-Pro-4-Amidinobenzylamid
   Ki _{Thrombin} 0,95 nM. Ki _{Trypsin} 61 nM, Ki _{Typsin}/Ki _{Thrombin} 64
Referenz: 4-Methoxy-benzensulfonyl-Lys-Pro-4-Amidinobenzylamid
   Ki _{Thrombin} 0,73 nM, Ki _{Trypsin} 82 nM, Ki _{Trypsin}/Ki _{Thrombin} = 112
*Kinetische Konstanten für den reversibel bindenden Inhibitor aus Beispiel 3*/*4* H-D-N(CH₂-CO-NH-PEG₅₀₀₀-OMe)Cha-Pro-4-(Amidino)Benzylamid
   Ki_{Thrombin}: 0,97 nM; Ki_{Trypsin}: 3,4 nM; Ki_{Trypsin}/Ki_{Thrombin}: 3,5
Referenz: H-D-N(CH₂-COOH)Cha-Pro-4-(Amidino)Benzylamid (Diese nicht PEG-gekoppelte Referenzverbindung ist identisch mit dem von der Firma Astra beschriebenen Inhibitor H317/86; Gustafsson et al., 1998 Thromb. Haemost. 79, 110-118).
   Ki_{Thombin}: 0,3 nM; Ki_{Trypsin}: 3,6 nM; Ki_{Trypsin}/Ki_{Thrombin}: 12

Bei den Inhibitoren dieses Typs führt die Beinführung der PEG-Kette zu einer leichten Verschlechterung der Thrombinhemmung, die Trypsinhemmung bleibt unbeeinflusst
*Kinetische Konstanten für den reversibel bindenden Inhibitor aus Beispiel 5 und 6*
H-D-N(CH₂-CO-NH-PEG₅₀₀₀-OMe)Cha-Pro-4-(Amidomethyl)-N(Amidino)Piperidin
Ki_{Thrombin}: 2,4 nM; Ki_{Trypsin}: 239 nM; Ki_{Trypsin}/Ki_{Thrombin}: 100
Referenz: H-D-N(CH₂-COOH)Cha-Pro-4-(Amidomethyl)-N(Amidino)Piperidin
Ki_{Thrombin}: 1,3 nM; Ki_{Trypsin}: 226 nM; Ki_{Trypsin}/Ki_{Thrombin}: 173

Auch bei den Inhibitoren dieses Typs führt die Einführung der PEG-Kette zu einer leichten Verschlechterung der Thrombinhemmung, die Trypsinhemmung bleibt dagegen nahezu unbeeinflusst.

### Kinetische Konstanten für den irreversibel bindenden Inhibitor aus Beispiel 7

Die folgenden kinetischen Konstanten wurden entsprechend dem in der Literatur beschriebenen Verfahren (Stein und Trainor, 1986 Biochemistry 25, 5414-5419) bestimmt.
H-d-N(CH₂-CONE-PEG₂₀₀₀-OMe)Cha-Pro-Arg-CH₂-Cl x 2 Trifluoressigsäure:
   Ki =10,6 nM; k_{inact} = 0,08 s⁻¹; k_{inact}/Kᵢ = 7,5 x 10⁶ M⁻¹s⁻¹
Als Referenz wurde die analoge, nicht PEG-gekoppelte Verbindung gemessen:
   Kᵢ = 3,8 nM; k_{inact} = 0,079 s⁻¹; k_{inact}/Kᵢ = 2,1 x 10⁷ M⁻¹s⁻¹
Aus der Literatur wurden die Daten für die Chlormethylketon-Leitstrukhu D-Phe-Pro-Arg-CH₂-Cl (PPACK) entnommen (Walker et al., 1985 Biochem. J. 230, 645-650):
   Kᵢ = 25 nM; k_{inact} = 0,11 s⁻¹; k_{inact}/Kᵢ = 4,4 x 10⁶ M⁻¹s⁻¹

Die PEG-gekoppelte Verbindung ist etwas weniger aktiv, als die analoge freie Verbindung, andererseits ist sie jedoch ein stärkerer Thrombinhemmstoff als das in der Literatur bereits beschriebene PACK.

### Beispiel 8a

Wirksame Thrombinhemmstoffe sollten bevorzugt die plasminolytischen Enzyme Plasmin, Urokinase und tPA kaum hemmen, da diese Proteasen an der Auflösung von Fibringerinnseln beteiligt sind und deshalb antithrombotische Eigenschaften besitzen. Deshalb wurden 2 ausgewählte Vertreter der in den Tabellen 1 und 2 beschriebenen Inhibitoren ohne PEG und ein PEG-gekoppelter Inhibitor bezüglich ihrer Hemmwirkung auf diese Enzyme untersucht.

Hemmkonstanten ausgewählter Inhibitoren bezüglich der Enzyme Thrombin, Plasmin, Urokinase und tPA (Werte in nM).

| Nr. | Sequenz | Kᵢ Thrombin | Kᵢ Plasmin | Kᵢ Urokinase | Kᵢ tPA |
|---|---|---|---|---|---|
| 1c | 4-MeO-bs-Gly-Pro-4-Amba | 0,97 | 5800 | 225000 | 630 |
| 50 | 4-MeO-bs-Asn-Pro-4-Amba | 0,36 | 17500 | 52000 | 47000 |
| | 4-MeO-bs-Asn(PEG₂₀₀₀-OMe)-Pro-4-Amba | 0,53 | 33500 | 157000 | 599000 |

Interessanterweise zeigt sich aus den Ki-Werten der in der obigen Tabelle aufgelisteten Inhibitoren, dass die Einführung einer geeigneten L-Aminosäure mit Seitenkette nicht nur eine Verstärkung der Thrombinhemmung bewirkt, sondern zusätzlich auch die Hemmung der fibrinolytischen Enzyme Plasmin und tPA abschwächt. Demzufolge sind die in der Tabelle aufgeführte Verbindung mit Asparagin und der PEG-gekoppelte Inhibitor sehr wirksame und gleichzeitig auch sehr selektive Thrombinhemmstoffe.

### Beispiel 9

### Eliminierung der Inhibitorkonjugate an Ratten

Vorbemerkungen: Für die Versuche wurden weibliche Wistarratten mit einem Körpergewicht zwischen 150-250 g verwendet. Die Tiere wurden unter konventionellen Bedingungen gehalten und erhielten Standardfutter und Trinkwasser ad libitum. Die Narkotisierung erfolgte mittels 1,5 g/kg Körpermasse Ethylurethan. Anschließend wurden die rechte und linke Vena jugularis präpariert und Katheter eingebunden, um zu unterschiedlichem Zeitpunkt Blut entnehmen zu können. Der Urin wurde über die gesamte Versuchsdauer gesammelt und die Inhibitorkonzentration mit einem Gerinnungstest bestimmt.

Abb. 5 zeigt die Eliminierung der in Formel (IV) dargestellten Verbindung nach intravenöser Gabe von 10 mg/kg an der Ratte. Die Eliminierungshalbwertszeit beträgt ca. 16.5 min.

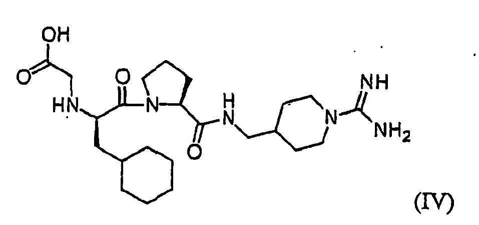

Abb. 6 zeigt die Ergebnisse der Bestimmung der kumulativen Wiederfindung im Urin der in Formel (IV) dargestellten Verbindung nach subkutaner Gabe an der Ratte. Es werden ca. 58 % der Verbindung im Urin wiedergefunden.

Zum Vergleich ist ein Beispiel mit einer analogen Verbindung angegeben, an die ein PEG5000 kovalent gekoppelt wurde.

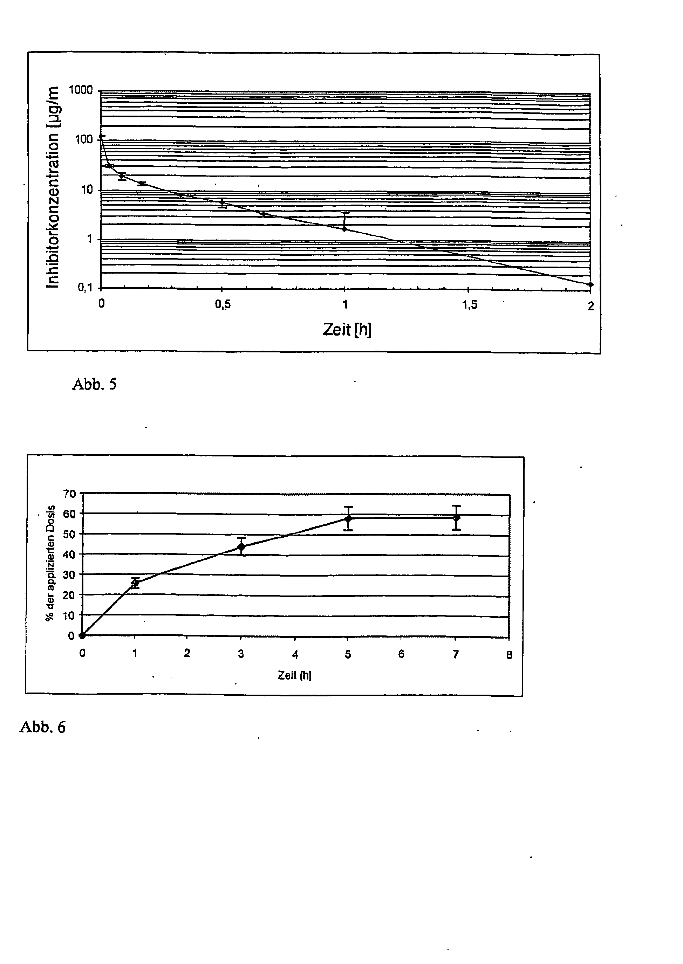

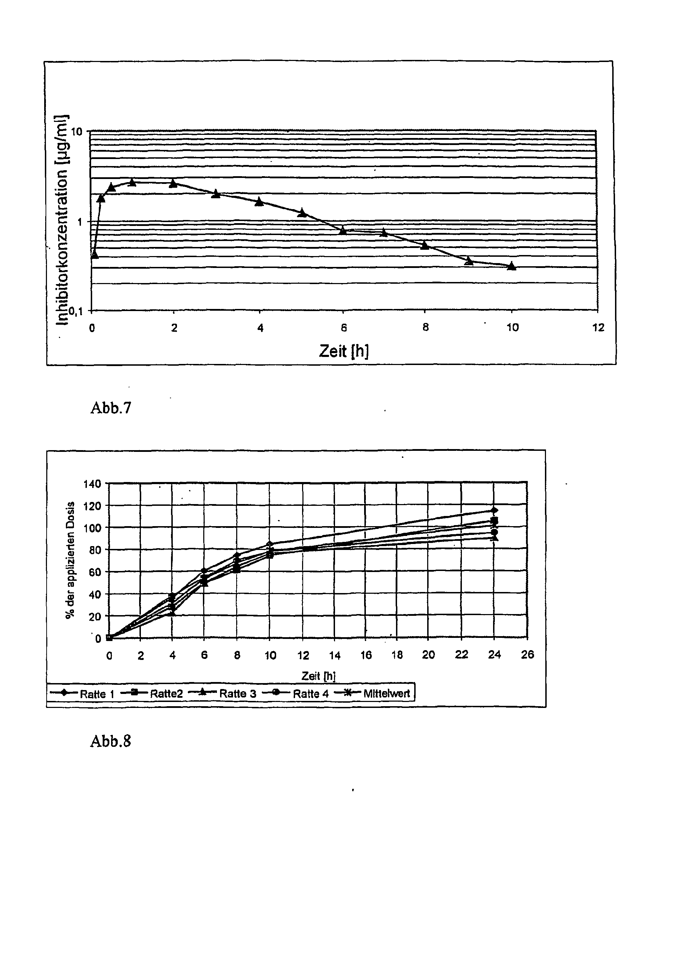

Abb. 7 zeigt den Blutspiegel der unten dargestellten Verbindung der Formel (V) (die PEG-Kette hat ein durchschnittliches Molekulargewicht von 5000 Da) nach subkutaner Gabe von 10 mg/kg. Die bestimmte Eliminierungshalbwertszeit beträgt ca. 2,6 Stunden und ist somit deutlich länger im Vergleich zur freien, nicht pegylierten Verbindung (siehe Abb. 5). 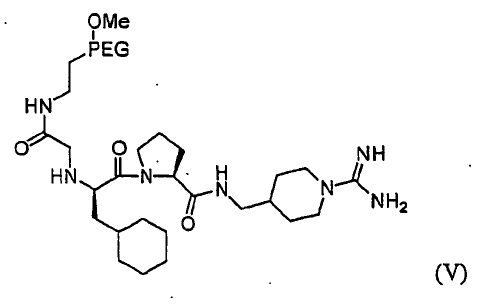 Abb.8 zeigt die Ergebnisse der Bestimmung der kumulativen Wiederfindung im Urin der Verbindung (V) nach subkutaner Gabe an der Ratte (n=4). Es werden nahezu 100 % der Verbindung im Urin wiedergefunden.

Abb. 9 zeigt eine halblogarithmische Darstellung der Konzentrations-Zeit-Kurve nach subkutaner Gabe von 5 mg/kg der Inhibitorstruktur (VI) an der Ratte. Die berechnete Eliminierungshalbwertszeit beträgt ca. 350 min. Die Halbwertszeit dieser Verbindung ist somit deutlich verlängert.

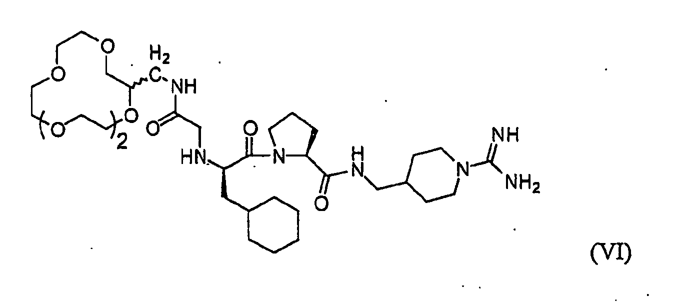

### Beispiel 10

### Eliminierung der Inhibitorkonjugate an Schweinen

Vorbemerkungen: Für die Versuche wurden weibliche und männliche Schweine mit einem Körpergewicht zwischen 12 und 15 kg verwendet. Die Narkotisierung der Tiere erfolgte durch intravenöse Gabe von 20 mg/kg Pentobarbital. In die linke oder rechte Vena jugularis wurde ein Katheter zur Blutentnahme und in die Luftröhre ein Trachealkatheter eingebunden, um eine gute Atmung der Tiere während der Versuche aufrechtzuerhalten. Die Blase der Tiere wurde ebenfalls mit einem Katheter versehen, so dass der Urin über die gesamte Versuchsdauer gesammelt werden konnte. Bei den männlichen Tieren wurde zusätzlich die Harnröhre abgebunden, so dass der Urin nur durch den Blasenkatheter in ein Sammelgefäß abfließen konnte. Die Inhibitorkonzentrationen wurden im Blut, im Plasma und im Urin mittels des Ecarin-Clotting-Tests (Nowak und Bucha, (1996) Quantitative determination of hirudin in blood and body fluids. Semin. Thromb. 22, 197-202) bestimmt.

Für die Eliminierungsuntersuchungen an Schweinen wurden die Inhibitoren der allgemeinen Formel (VII) mit einer durchschnittlichen PEG-Kettenlänge von 2000 oder 10000 Da verwendet.

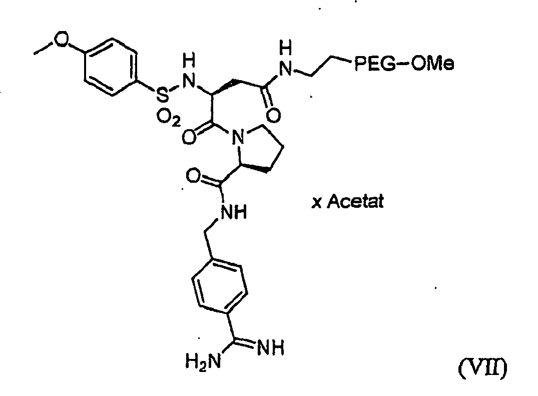

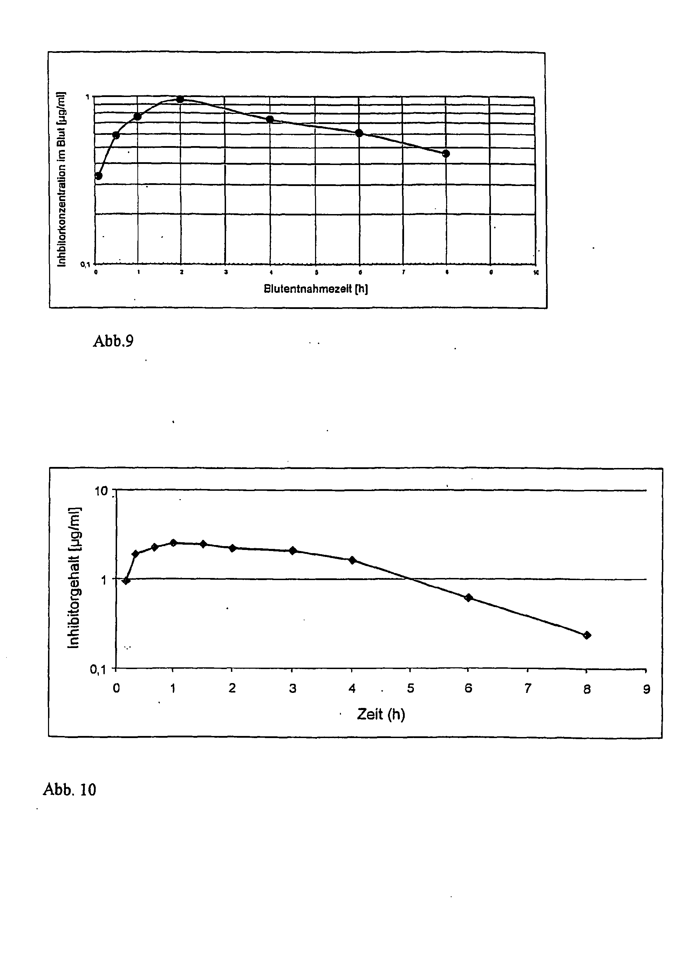

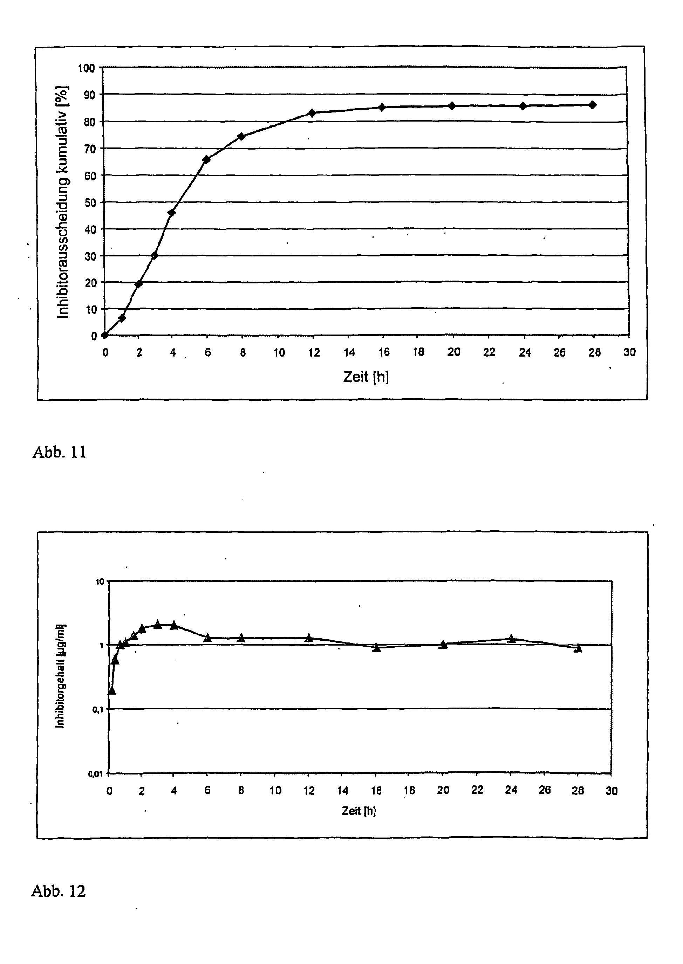

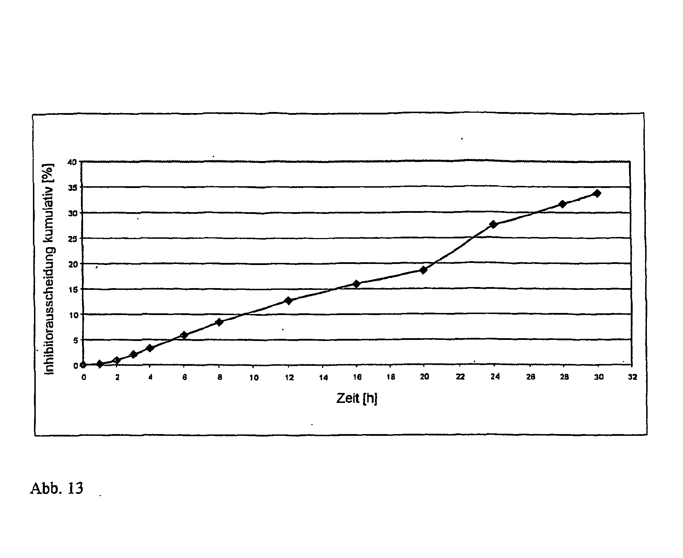

In den Untersuchungen zeigte sich überraschenderweise, dass in Abhängigkeit der Länge der PEG-Kette der Inhibitoren der allgemeinen Formel (VII) große Unterschiede in der Eliminierungsgeschwindigkeit der Inhibitoren aus dem Blut auftreten. Im Falle der Inhibitoren mit einer durchschnittlichen PEG-Kettenlänge um 10000 Da (Abb. 12 und 13) konnten bis zu 28 h nach subkutaner Inhibitorgabe lang anhaltende, antithrombotisch wirksame Blutspiegel gemessen werden. Interessanterweise wurden bis zu diesem Zeitpunkt nur ca. 35 % des Inhibitors im Urin wiedergefunden.
Im Gegensatz dazu wurde der Inhibitor der allgemeinen Formel (VII) mit einer durchschnittlichen PEG-Kettenlänge von 2000 Da bereits nach ca. 12 h nahezu vollständig über die Niere eliminiert (Abb. 10 und 11).

Abb. 10 zeigt die Plasmaspiegel des Inhibitors der allgemeinen Struktur (VII) mit einer durchschnittlichen PEG-Kette um 2000 Da nach subcutaner Gabe von 2,5 mg/kg in Schweinen.

Abb. 11 zeigt die kumulative Ausscheidung des Inhibitors der allgemeinen Struktur (VII) mit einer durchschnittlichen PEG-Kette um 2000 Da nach subcutaner Gabe von 2,5 mg/kg im Urin von Schweinen.

Abb. 12 zeigt die Plasmaspiegel des Inhibitors der allgemeinen Struktur (VII) mit einer durchschnittlichen PEG-Kette um 10000 Da nach subcutaner Gabe von 20 mg/kg in Schweinen.

Abb. 13 zeigt die kumulative Ausscheidung des Inhibitors der allgemeinen Struktur (VII) mit einer durchschnittlichen PEG-Kette um 10000 Da nach subcutaner Gabe von 20 mg/kg im Urin von Schweinen.

## Patentansprüche

1. Verbindung der allgemeinen Struktur (I) 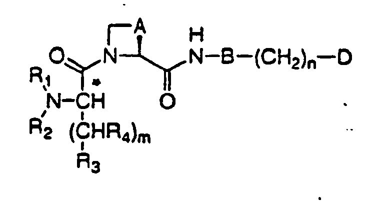 in der
n 0, 1, 2, 3, 4 oder 5 ist;
A eine Ethylen
B eine Bindung oder 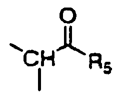 ist, wobei R₅ ein Alkylrest ist der 1 bis 4, bevorzugt 1 oder 2 und besonders bevorzugt ein C-Atom umfasst und mit einem oder mehreren gleichen oder unterschiedlichen
Halogenatomen oder mit einem Rest 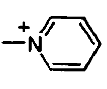 substituiert sein kann;
D einen Rest 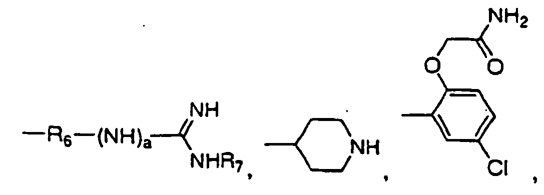 -R₈-(CH₂)_{b}-NH₂, oder 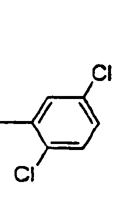 darstellt, wobei a 0 oder 1 und b 0, 1 oder 2 ist und R₆ und R₈ unabhängig voneinander bivalente Reste sind, ausgewählt aus aromatischen oder gesättigten 6-Ringen, die neben Kohlenstoff noch ein Heteroatom enthalten können und einen oder mehrere gleiche oder unterschiedliche Alkylsubstituenten tragen können, und R₆ darüberhinaus -NH- sein kann falls a 0 ist, R₇ ein Wasserstoffatom oder -NH₂ ist;
R₁ ein Wasserstoffatom oder ein Arylsulfonyl-, Aralk-ylsulfonyl-, Cycloalkylmethylsulfonyl-, Cycloalkylethylsulfonyl- oder ein Alkylsulfonylrest ist, deren Arylteil gegebenenfalls einen, zwei, drei, vier oder fünf Substituenten, die unabhängig voneinander ausgewählt sind aus Halogenatomen, Alkyl- oder Alkoxyresten, tragen kann, oder mit einem weiteren Arylrest verknüpft ist;
und entweder
R₂ -(CH₂)ₚ-CO-NH-(CH₂)_{q}-X, -(CH₂)ᵣ-NH-CO-(CH₂)ₛ-V-X oder
-(CH₂)ᵣ-NH-CO-O-(CH₂)ₛ-V-X ist
wobei
p eine ganze Zahl von 1 bis 5 und q eine ganze Zahl von 2 bis 5 ist,
r eine ganze Zahl von 2 bis 5 und s ist eine ganze Zahl von 1-5 ist,
V eine Bindung oder -CO-NH-(CH₂)_{c}- und c eine ganze Zahl von 2 bis 5 ist, und
X ein Oligo- oder Polyalkylenglykol der Struktur -[O-(CH₂)_{d}]_{c}-OZ oder -[O-CH(CH₃)-CH₂]_{c}-OZ oder einen Cyclus der Struktur 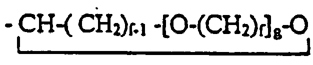 darstellt und d
eine ganze Zahl von 2 bis 6, e eine ganze Zahl von 3 bis 1000 ist, Z ein Wasserstoffatom oder ein Alkylrest ist oder die gesamte Inhibitorstruktur repliziert, die an die freie Valenz des Restes X gebunden ist, f eine ganze Zahl von 2 bis 6 ist und g eine ganze Zahl von 3 bis 10, ist; und
R₃ ein Phenyl- oder Cyclohexylrest ist, der mit 1 bis 5 gleichen oder unterschiedlichen Substituenten, unabhängig voneinander ausgewählt aus Halogenatomen, Allyl- oder Alkoxyresten oder Hydroxylgruppen substituiert sein kann und
R₄ ein Wasserstoffatom, Phenyl- oder Cyclohexylrest ist, der mit 1 bis 5 gleichen oder unterschiedlichen Substituenten, unabhängig voneinander ausgewählt aus Halogenatomen, Alkyl- oder Alkoxyresten oder Hydroxylgruppen substituiert sein kann,
m 0, 1, 2, 3 oder 4 ist und
am mit * gekennzeichneten Kohlenstoffatom eine D-Konfguration vorliegt;
oder
R₂ ein Wasserstoffatom ist und
R₃ -CO-NH-(CH₂)_{q}-X, -CO-W₁-W₂-(CH₂)_{q}-X, -NH-CO-(CH₂)ₛ-V-X, -NH-CO-O-(CH₂)ₛ-V-X, -S-CH₂-CO-NH-(CH₂)ₜ-X oder -S-S-CH₂-CH₂-X ist,
wobei q, s=X und V wie vorstehend definiert sind und t eine ganze Zahl von 2 bis 5 ist, und der Rest W₁ -O- oder -NH- ist, und W₂ eine Bindung darstellt oder die Struktur -(CH₂)ᵥ-Ph. (CH₂)_{v'}-Amid- aufweist, in der und v' unabhängig von einander 0, 1 oder 2 sind, Ph einen 1,2-, 1,3- oder 1,4-substituierten Phenylrest und Amid -HN-(O)C- oder -C(O)NH- darstellt, und
R₄ ein Wasserstoffatom ist
m 1, 2, 3, 4 oder 5 ist und
am mit gekennzeichneten Kohlenstoffatom eine L-Konfiguration vorliegt.

2. Verbindung gemäß Anspruch 1, wobei B eine der folgenden Strukturen darstellt

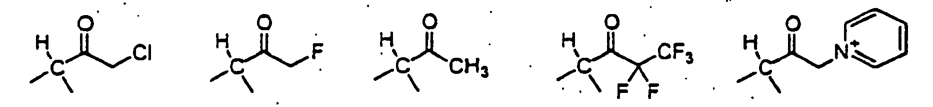

3. Verbindung gemäß Anspruch 1 oder 2, wobei D eine der folgenden Strukturen darstellt

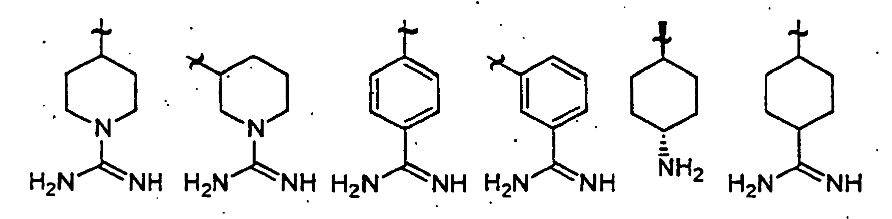

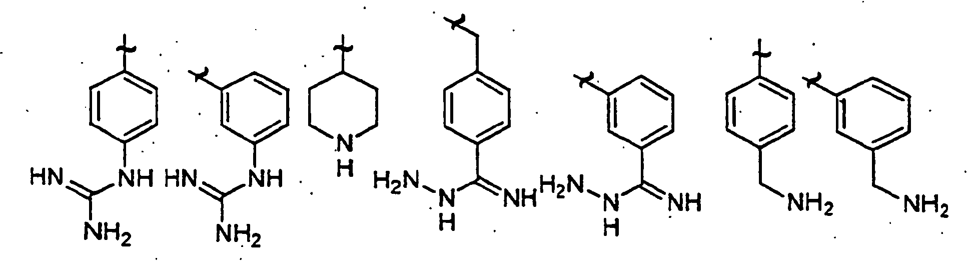

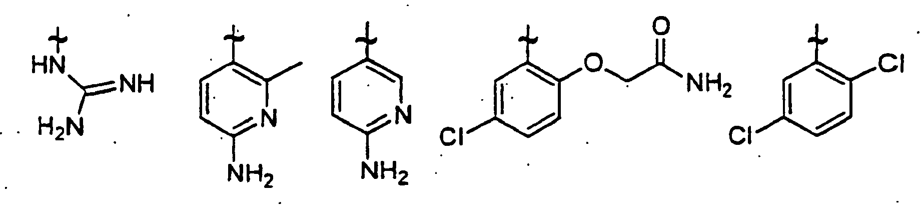

4. Verbindung gemäß einem der Ansprüche 1 bis 3, wobei X eine cyclische Struktur der Formel 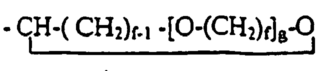 aufweist und f und g wie in Anspruch1 definiert sind

5. Verbindung gemäß Anspruch 1 mit einer der folgenden Formeln Ia-Ih 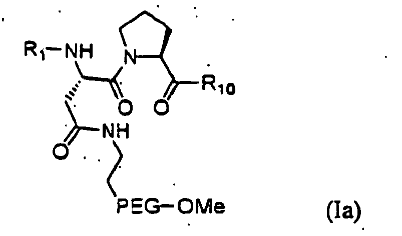 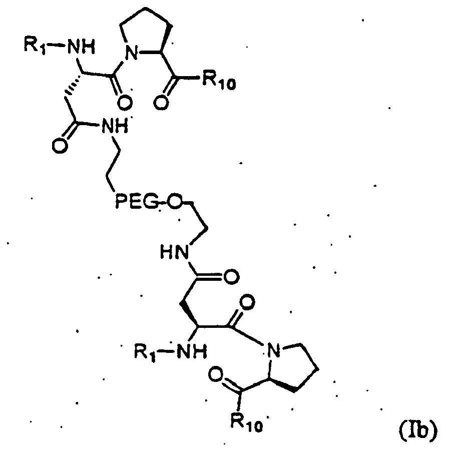 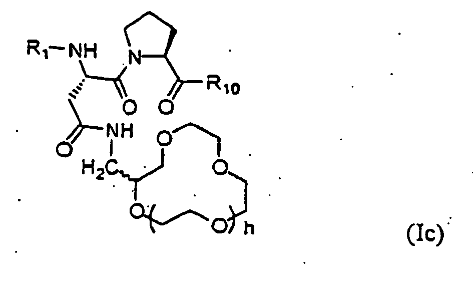 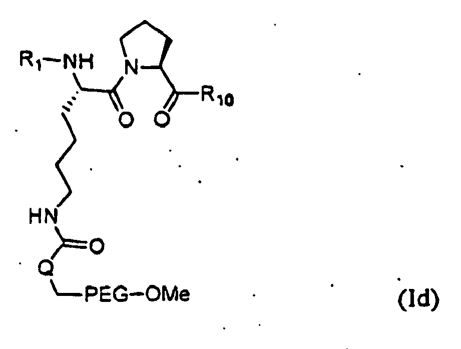 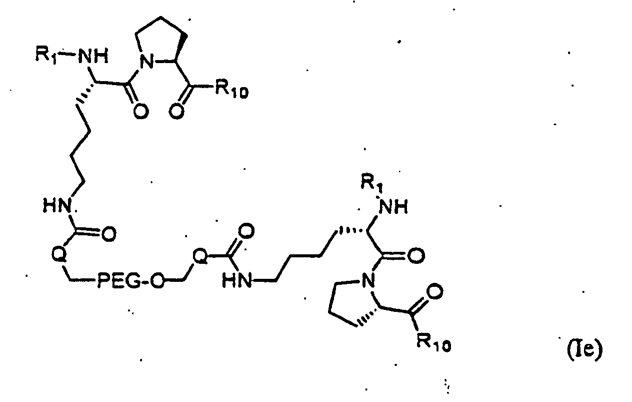 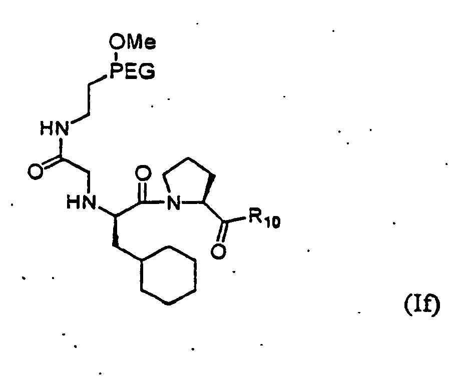 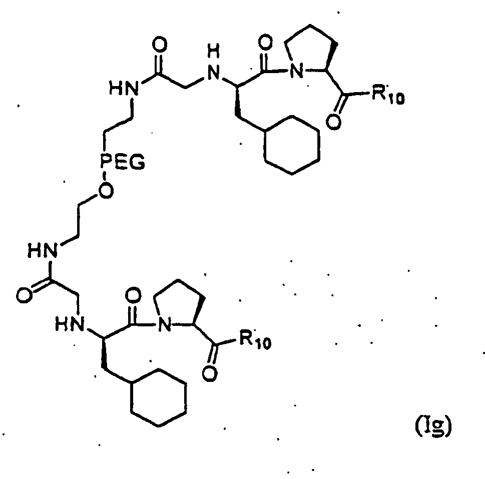 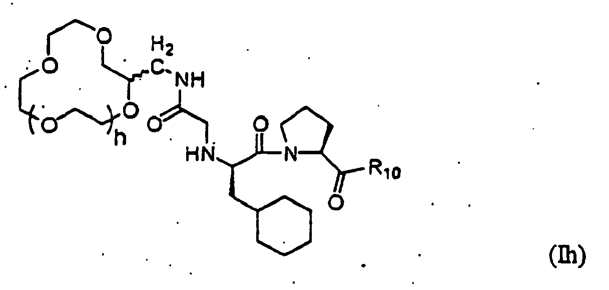 in denen R₁ ein 4-Methoxy-phenylsulfonyl-, 4-Methoxy-3-Chlor-phenylsulfonyl-, 4-Methon-3-Methyl-phenylsulfonyl- oder 4-Methoxy-2,3,6-trunethyl-phenylsulfonylrest ist, Q -(CH₂)s'- mit s' = 0, 1, 2, 3 oder 4, -O-CH₂- oder -CH₂-CH₂-CO-NH-CH₂ ist
und
PEG eine Polyethylenglykolstruktur der Formel -[O-C₂H₄]ᵢ- darstellt, mit Werten für i von 3 bis 1000,
h für 1, 2, 3 oder 4 steht
und R₁₀ 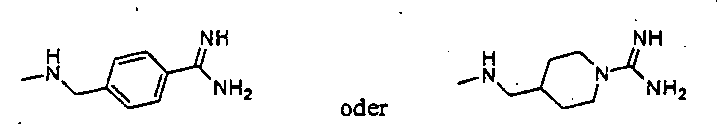 ist.

6. Verwendung einer Verbindung gemäß einem der Ansprüche 1 bis 5 als Wirkstoff bei der Herstellung eines Medikaments zur Therapie oder Prophylaxe thrombotischer Komplikationen, wobei der Wirkstoff überwiegend renal eliminiert wird.

7. Verwendung einer Verbindung gemäß einem der Ansprüche 1 bis 5 zum Beschichten einer Oberfläche.

8. Verfahren zur Modifikation einer Oberfläche, umfassend das in Kontakt bringen der Oberfläche mit einer Verbindung gemäß einem der Ansprüche 1 bis 5.

## Claims

1. Compound of the general structure (I) 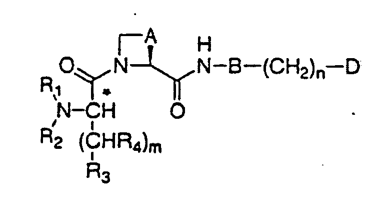 wherein
n is 0, 1, 2, 3, 4 or 5;
A is an ethylene group;
B is a bond or 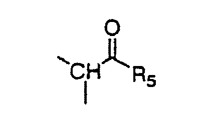 wherein R₅ is alkyl comprising 1 to 4, preferably 1 or 2 and especially preferred one carbon atom, and can be substituted with one or more identical or different halogen atoms
or with a group 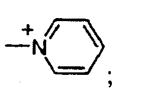 D represents a group 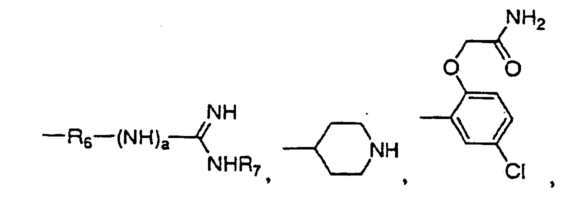 -R₈-(CH₂)_{b}-NH₂, or 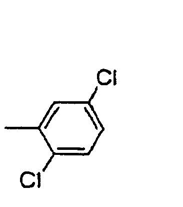 wherein a is 0 or 1, b is 0, 1 or 2, and R₆ and R₈ are independently bivalent groups selected from aromatic or saturated 6-membered rings which can comprise a heteroatom in addition to carbon and can carry one or more identical or different alkyl substituents, and wherein R₆ can furthermore be -NH- if a is 0, R₇ is a hydrogen atom or -NH₂;
R₁ is a hydrogen atom or an arylsulfonyl, aralkylsulfonyl, cycloalkylmethylsulfonyl, cycloalkylethylsulfonyl or alkylsulfonyl group, the aryl portion of which can optionally carry one, two, three, four or five substituents independently selected from halogen atoms, alkyl or alkoxy groups or is linked with another aryl,
and either:
R₂ is -(CH₂)ₚ-CO-NH-(CH₂)_{q}-X, -(CH2₎ᵣ-NH-CO-(CH₂)ₛ-V-X or -(CH₂)ᵣ-NH-COO-(CH₂)ₛ-V-X
wherein
p is an integer from 1 to 5 and q is an integer from 2 to 5;
r is an integer from 2 to 5 and s is an integer from 1 to 5;
V is a bond or -CO-NH-(CH₂)_{c}- and c is an integer from 2 to 5; and
X is an oligo or polyalkylene glycol with the structure -[O-(CH₂)_{d}]ₑ-OZ or -[O-CH(CH₃)-CH₂]ₑ-OZ or a cycle with the structure 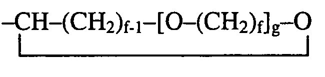 and d is an integer from 2 to 6, e is an integer from 3 to 1,000, Z is a hydrogen atom or alkyl or replicates the entire inhibitor structure bonded to the free valence of the group X,
f is an integer from 2 to 6 and g is an integer from 3 to 10; and
R₃ is a phenyl or cyclohexyl group which can be substituted with 1 to 5 identical or different substituents independently selected from halogen atoms, alkyl or alkoxy or hydroxyl groups and
R₄ is a hydrogen atom, phenyl or cyclohexyl group which can be substituted with 1 to 5 identical or different substituents independently selected from halogen atoms, alkyl or
alkoxy or hydroxyl groups,
m is 0, 1, 2, 3 or 4 and
a D-configuration is present at the carbon atom marked with *;
or
R₂ is a hydrogen atom and
R₃ is -CO-NH-(CH₂)_{q}-X, -CO-W₁-W₂-(CH₂)_{q}-X, -NH-CO-(CH₂)ₛ-V-X, -NH-COO-(CH₂)ₛ-V-X, -X-CH₂-CO-NH-(CH₂)ₜ-X or -S-S-CH₂-CH₂-X,
wherein q, s, X and V are as defined above and t is an integer from 2 to 5, and the group W₁ is -0- or -NH- and W₂ represents a bond or has the structure -(CH₂)ᵥ-Ph-(CH₂)_{v'}-amide-, wherein v and v' are independently 0, 1 or 2, Ph represents a 1,2-, 1,3- or 1,4-substituted phenyl group and amide represents -HN-(O)C- or -C(O)NH-,
and R₄ is a hydrogen atom,
m is 1, 2, 3, 4 or 5 and
an L-configuration is present at the carbon atom marked with *.

2. Compound according to claim 1, wherein B represents one of the following structures

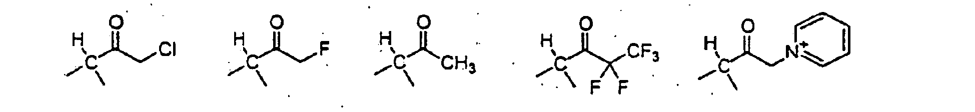

3. Compound according to claim 1 or. 2, wherein D represents one of the following structures

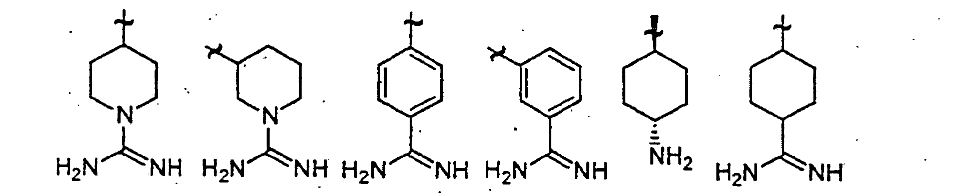

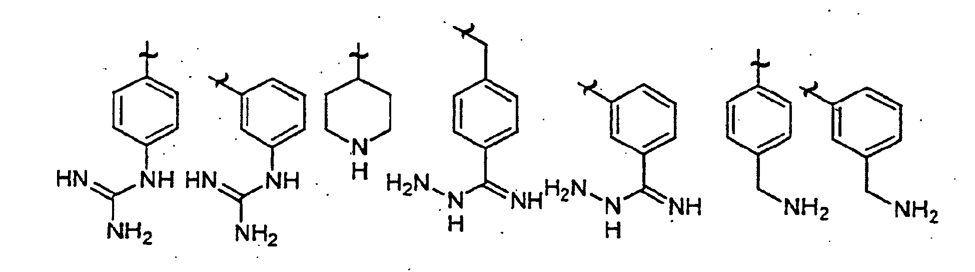

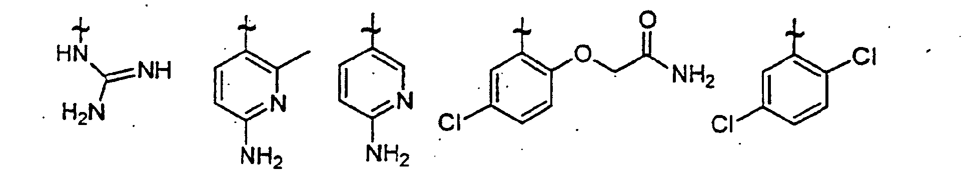

4. Compound according to any one of claims 1 to 3, wherein X has a cyclic structure of the formula 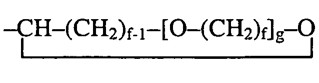 and f and g are as defined in claim 1.

5. Compound according to claim 1 having one of the following formulas Ia to Ih 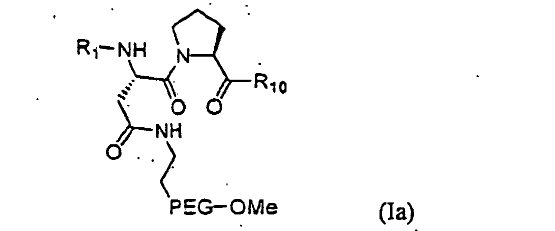 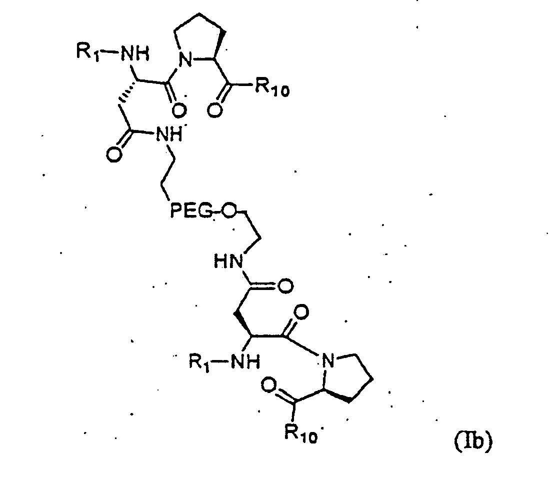 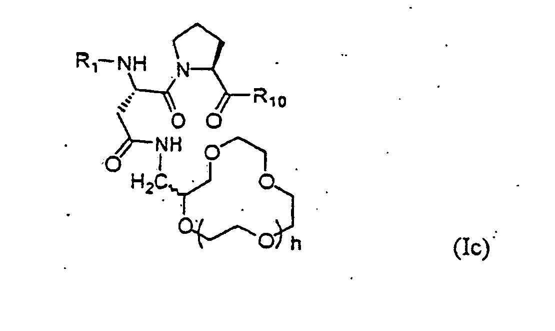 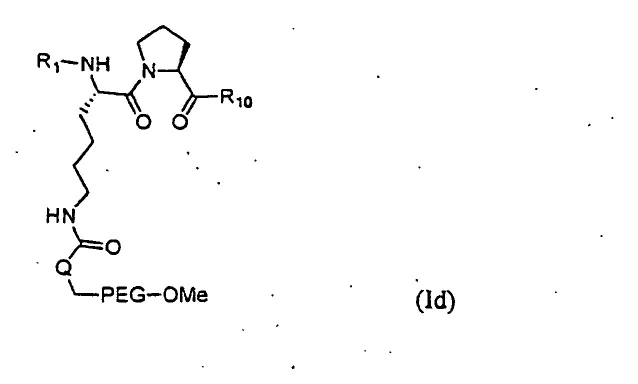 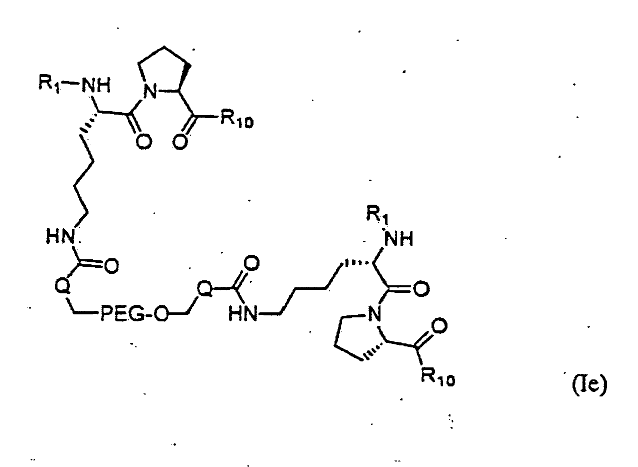 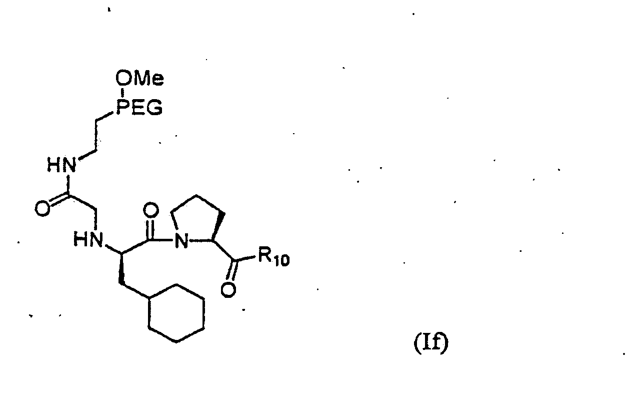 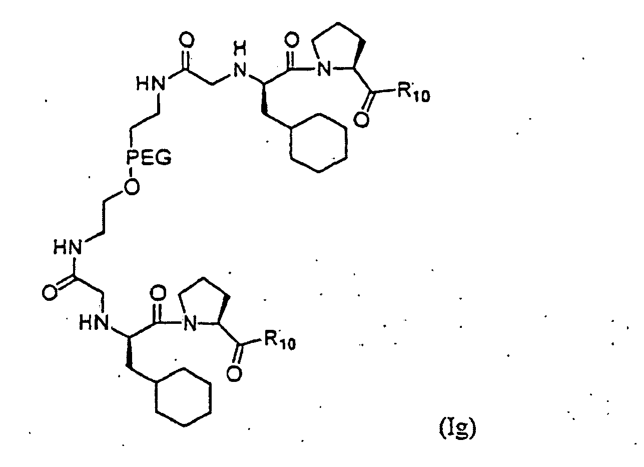 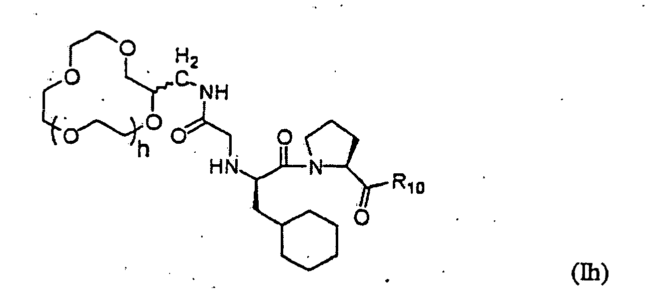 wherein R₁ is a 4-methoxy-phenylsulfonyl, 4-methoxy-3-chloro-phenylsulfonyl, 4-methoxy-3-methyl-phenylsulfonyl or 4-methoxy-2,3,6-trimethyl-phenylsulfonyl group, Q is -(CH₂)s'- wherein s' = 0, 1, 2, 3, or 4, -O-CH₂- or -CH₂-CH₂-CO-NH-CH₂, and PEG is a polyethylene glycol structure of the formula -[O-C₂H₄]ᵢ-, wherein i is 3 to 1,000,
h represents 1, 2, 3 or 4
and R₁₀ is

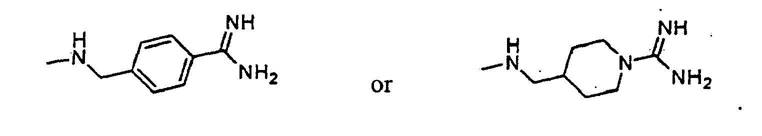

6. Use of a compound according to any one of claims 1 to 5 as an active ingredient in the preparation of a medicament for therapy or prophylaxis of thrombotic complications, said active ingredient being predominantly eliminated renally.

7. Use of a compound according to any one of claims 1 to 5 for coating a surface.

8. Process for modifying a surface, comprising contacting the surface with a compound according to any one of claims 1 to 5.

## Revendications

1. Composé de structure générale (I) 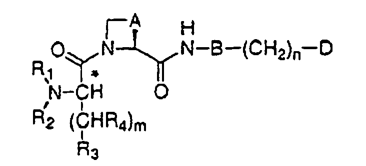 où n est égal à 0, 1, 2, 3, 4 ou 5 ; A est un groupe éthylène ; B est une liaison ou 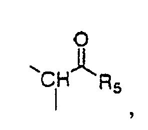 R₅ étant un résidu alkyle comprenant de 1 à 4, avantageusement 1 ou 2 et de préférence un seul, atome(s) de C, et pouvant être substitué par un ou plusieurs atomes d'halogène identiques ou différents ou par un résidu 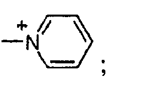 où D représente un résidu 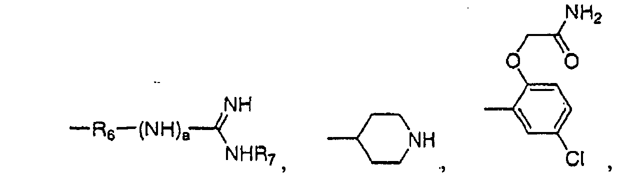 -R₈-(CH₂)_{b}-NH₂ ou 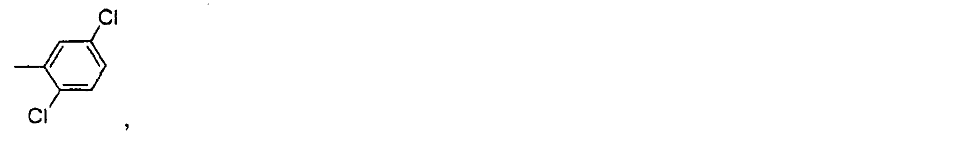 a étant égal à 0 ou à 1 et b à 0, à 1 ou à 2, et R₆ et R₈ étant des résidus bivalents indépendants l'un de l'autre, sélectionnés parmi les cycles à 6 chaînons aromatiques ou saturés pouvant contenir un hétéroatome en plus du carbone et porter un ou plusieurs substituants alkyles identiques ou différents, et R₆ pouvant en outre être -NH- si a est égal à 0, R₇ étant un atome d'hydrogène ou -NH₂ ;
où R₁ est un atome d'hydrogène ou un résidu arylsulfonyle, aralkylsulfonyle, cycloalkylméthylsulfonyle, cycloalkyléthylsulfonyle ou alkylsulfonyle, dont la partie aryle peut le cas échéant porter un, deux, trois, quatre ou cinq substituants sélectionnés indépendamment les uns des autres parmi les atomes d'halogène, les résidus alkyles ou alkoxyles, ou est liée à un autre résidu aryle ;
et où soit
R₂ est -(CH₂)ₚ-CO-NH-(CH₂)_{q}-X, -(CH₂)ᵣ-NH-CO-(CH₂)ₛ-V-X ou -(CH₂)ᵣ-NH-CO-O-(CH₂)ₛ-V-X,
p étant un nombre entier de 1 à 5 et q un nombre entier de 2 à 5,
r étant un nombre entier de 2 à 5 et s un nombre entier de 1 à 5,
V étant une liaison ou -CO-NH-(CH₂)_{c}-, et c un nombre entier de 2 à 5, et
où X représente un oligo- ou polyalkylèneglycol de structure -[O-(CH₂)_{d}]ₑ-OZ ou -[O-CH(CH₃)-CH₂]ₑ-OZ, ou un cycle de structure 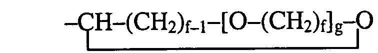 et d un nombre entier de 2 à 6, e un nombre entier de 3 à 1000, Z est un atome d'hydrogène ou un résidu alkyle ou reproduit l'ensemble de la structure d'inhibiteur qui est liée à la valence libre du résidu X, f est un nombre entier de 2 à 6 et g un nombre entier de 3 à 10 ; et
où R₃ est un résidu phényle ou cyclohexyle, pouvant être substitué par 1 à 5 substituants identiques ou différents, sélectionnés indépendamment les uns des autres parmi les atomes d'halogène, les résidus alkyles ou alkoxyles ou les groupes hydroxyles et
R₄ est un atome d'hydrogène, un résidu phényle ou cyclohexyle, pouvant être substitué par 1 à 5 substituants identiques ou différents, sélectionnés indépendamment les uns des autres parmi les atomes d'halogène, les résidus alkyles ou alkoxyles ou les groupes hydroxyles,
m est égal à 0, 1, 2, 3 ou 4 et
où une configuration D est présente sur l'atome de carbone identifié par * ;
soit
R₂ est un atome d'hydrogène et
R₃ est -CO-NH-(CH₂)_{q}-X, -CO-W₁-W₂-(CH₂)_{q}-X, -NH-CO-(CH₂)ₛ-V-X, -NH-CO-O-(CH₂)ₛ-V-X, -S-CH₂-CO-NH-(CH₂)ₜ-X ou -S-S-CH₂-CH₂-X, q, s, X et V étant définis comme ci-dessus et t étant un nombre entier de 2 à 5, et le résidu W₁ étant -0- ou -NH-, et W₂ représentant une liaison ou la structure -(CH₂)ᵥ-Ph-(CH₂)ᵥ,-amide-, dans laquelle v et v' sont 0, 1 ou 2 indépendamment l'un de l'autre, Ph un résidu phényle substitué en 1,2, 1,3 ou 1,4 et où amide représente -HN-(O)C- ou -C(O)NH-, et
où R₄ est un atome d'hydrogène,
m est égal à 1, 2, 3, 4 ou 5 et
où une configuration L est présente sur l'atome de carbone identifié par *.

2. Composé selon la revendication 1, où B représente une des structures suivantes :

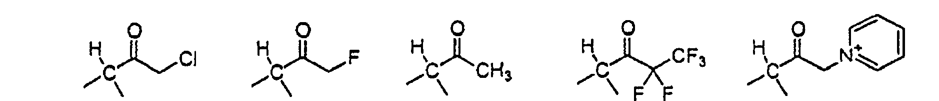

3. Composé selon la revendication 1 ou 2, où D représente une des structures suivantes :

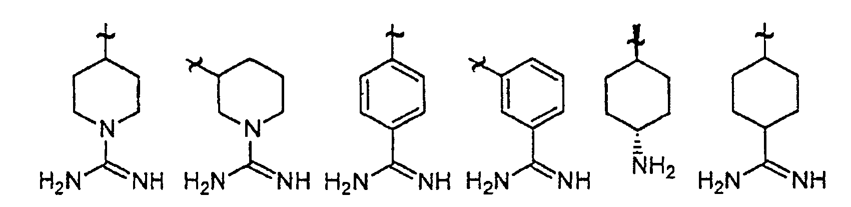

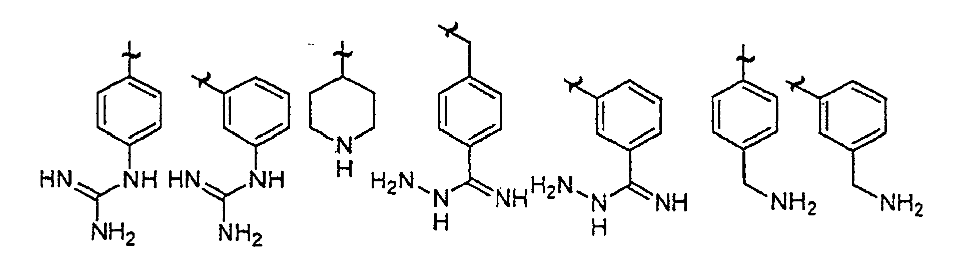

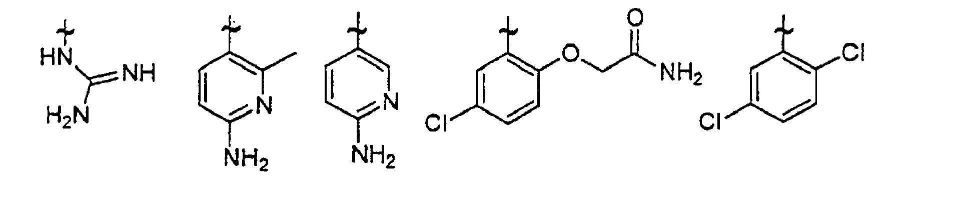

4. Composé selon l'une des revendications 1 à 3, où X présente une structure cyclique de formule 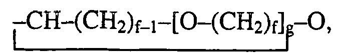 et où f et g sont définis comme dans la revendication 1.

5. Composé selon la revendication 1 avec une des formules Ia à Ih suivantes : 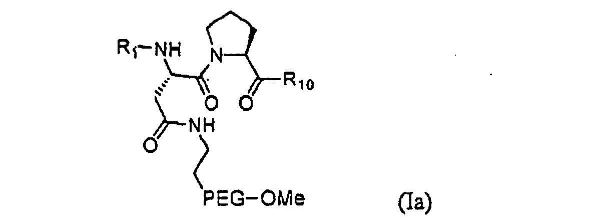 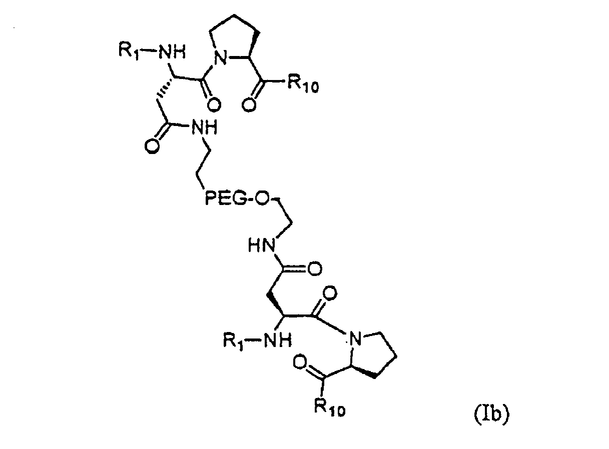 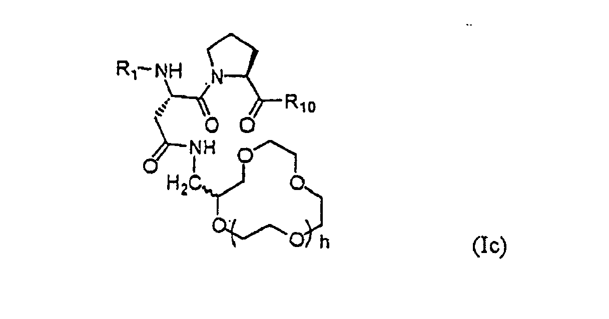 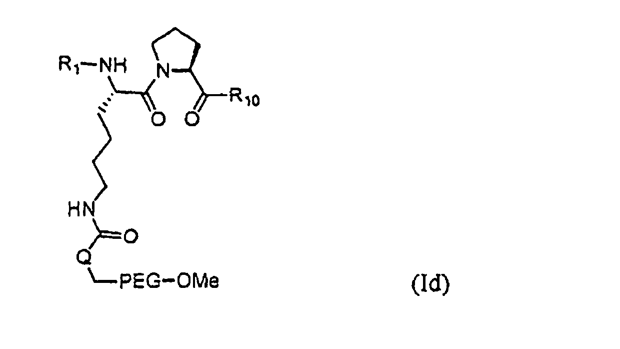 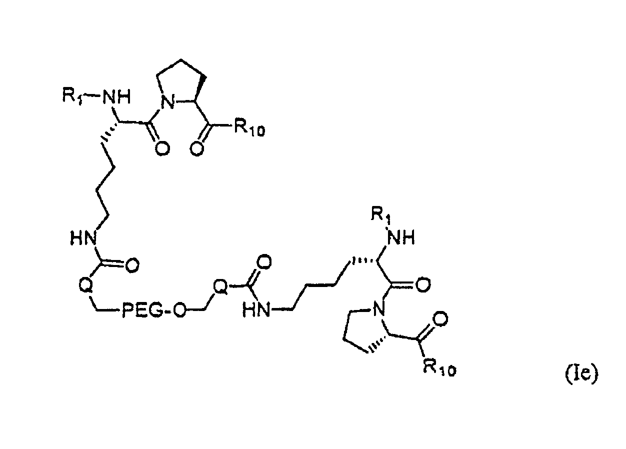 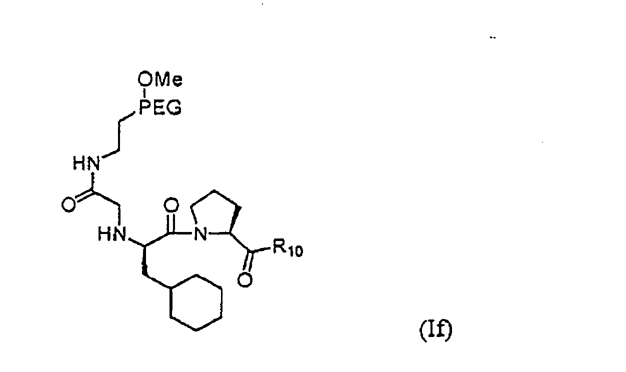 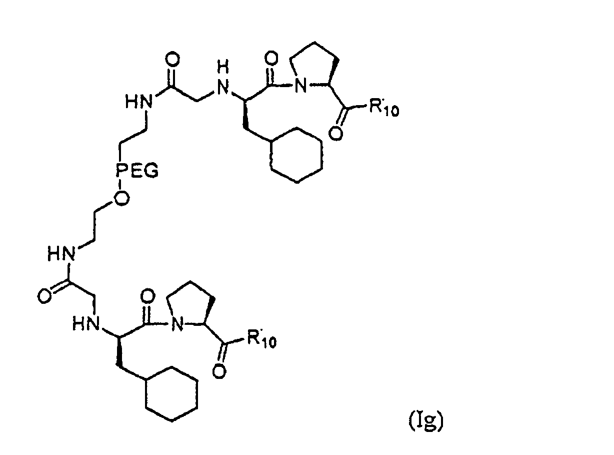 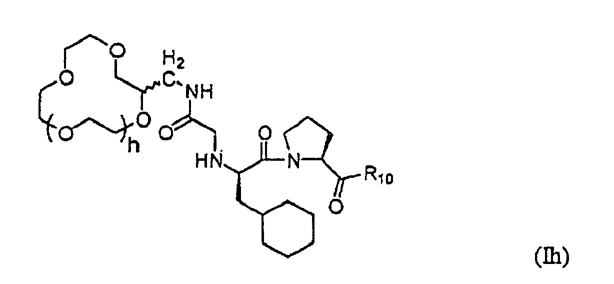 où R₁ est un résidu 4-méthoxy-phénylsulfonyle, 4-méthoxy-3-chlorophénylsulfonyle, 4-méthoxy-3-méthyl-phénylsulfonyle ou 4-méthoxy-2,3,6-triméthyl-phénylsulfonyle, Q est -(CH₂)s'- avec s' = 0, 1, 2, 3 ou 4, -O-CH₂- ou -CH₂-CH₂-CO-NH-CH₂
et
où PEG représente une structure polyéthylèneglycol de formule -[O-C₂H₄]ᵢ-, avec des valeurs comprises de 3 à 1000 pour i,
où h représente 1, 2, 3 ou 4 et
où R₁₀ est :

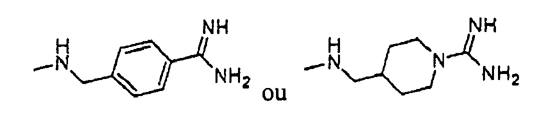

6. Utilisation d'un composé selon l'une des revendications 1 à 5 en tant que principe actif pour la fabrication d'un médicament destiné au traitement ou à la prophylaxie de complications thrombotiques, ledit principe actif étant majoritairement éliminé par voie rénale.

7. Utilisation d'un composé selon l'une des revendications 1 à 5 pour le revêtement d'une surface.

8. Procédé de modification d'une surface, comprenant la mise en contact de la surface avec un composé selon l'une des revendications 1 à 5.
